# EUROPEAN PATENT APPLICATION

(11) **EP 2 380 566 A2**
(43) Date of publication of application: **26.10.2011**
(21) Application number: 11171275.8
(22) Date of filing: 14.09.2007
(51) Int. Cl.: A61K 31/015, A61K 31/19, A61P 3/06, A61P 3/08, A61P 3/10

(54) **Treatment of insulin resistance or diseases associated with insulin resistance using steviol or isosteviol**

(30) Priority: 15.09.2006 DK 200601200; 06.09.2007 DK 200701274
(62) Divisional of application: 07801392.7
(71) Applicant: Stevia APS, 2820 Gentofte (DK)
(72) Inventor: Hermansen, Kjeld, 8250 Egå (DK); Jeppesen, Per Bendix, 8250 Egå (DK)
(74) Representative: Høiberg A/S

(57) **Abstract**

The present invention relates to the use of a substance with the core structure of formula (I), or pharmaceutically acceptable salts, solvates or prodrugs thereof, for the manufacture of a composition for the treatment of insulin resistance or diseases associated with insulin resistance, preferably in human subject in a daily dosage in a range of from about 5 mg to about 1500 mg. The invention furthermore relates to a method of treating insulin resistance or diseases associated with insulin resistance in a mammal, said method comprises administering to said mamma, preferably a human subject, a substance with the core structure of formula (I), preferably in a daily dosage in a range from about 5 mg to about 1500 mg. The substance is preferably isosteviol or steviol, or pharmaceutically acceptable salts, solvates or prodrugs thereof. Examples of diseases associated with insulin resistance are e.g. type 2 diabetes mellitus, insulin resistance syndrome, impaired glucose tolerance, the metabolic syndrome, hyperglycemia, hyperinsulinemia, arteriosclerosis, hypercholesterolemia, hypertriglyceridemia, hyperlipidemia, dyslipidemia, obesity, central obesity, polycystic ovarian syndrome, hypercoagulability, hypertension, microalbuminuria, or any combinations thereof.

## Description

### Field of invention

The present invention relates to the use of steviol and/or isosteviol for the treatment of diseases associated with insulin resistance.

### Background of invention

In a person with normal metabolism, insulin is released from the beta cells of Islets of Langerhans located in the pancreas in response to an elevated blood glucose level, allowing glucose to enter insulin-sensitive tissues and hereby maintain normal blood glucose levels. Diabetes has become the fourth leading cause of death in most developed countries and will be one of the most challenging health problems worldwide in the 21 st century. There are two major forms of diabetes: Type-1 diabetes is characterised by the inability to synthesise insulin, whereas in Type-2 diabetes the body becomes resistant to the effects of insulin and the beta cell dysfunction exercising increased basal insulin secretion but impaired glucose stimulated insulin secretion. In an "insulin resistant" individual the body is less sensitive to insulin levels in the blood, and hence the metabolic activities triggered by insulin as seen in normal individuals do not proceed or proceed at lower levels. This leads to a condition in which normal amounts of insulin are inadequate to produce a normal insulin response from fat, muscle and liver cells i.e. the cells are not able to absorb glucose and other nutrients.

As a result of the lowered metabolic response, the normal physiological feedback mechanisms cause the beta cells to increase insulin production to compensate for the insensitivity of the response to insulin. As the insulin response continues to decrease, insulin production continues to increase. However, sustained insulin resistance weakens the beta cells and gradually degrades the insulin secretion capacity, thus proceeding to a more pronounced diabetic stage.

The inability of the β-cells to produce more insulin in a condition of hyperinsulinemia is what characterizes the transition from insulin resistance to Type 2 diabetes (see McGarry: "Dysregulation of Fatty Acid Metabolism in the Etiology of Type 2 Diabetes"; Diabetes (2002); 51 (1); 7-18). Thus, the onset of resistance to insulin may serve as an indicator of an eventual diabetic disease in an individual. It should in this respect be noted, that insulin resistance is observed not only in respect of diabetic patients but also in disorders caused by abnormalities in lipid metabolism such as arteriosclerosis, etc. (see Saltiel: "New Perspectives into the Molecular Pathogenesis and Treatment of Type 2 Diabetes"; Cell; (2001); 104; 517-529). Insulin resistance is also a part of the etiology of hypertension, hyperlipidemia and obesity, in addition to arteriosclerosis and diabetes.

As a matter of fact, insulin resistance is often found in people with visceral adiposity, hypertension, glucose intolerance and dyslipidemia involving elevated triglycerides, small dense low-density lipoprotein (sdLDL) particles, and decreased HDL cholesterol levels. Insulin resistance is furthermore, often associated with a hypercoagulable state (impaired fibrinolysis) and increased inflammatory cytokine levels.

While the mechanism of insulin resistance largely remains unknown, a large number of factors can contribute to insulin resistance. Insulin resistance and beta cell dysfunction are the primary abnormalities in Type-2 diabetes, where the capacity for beta cell growth also contributes to the defects. A deficient beta cell proliferating capacity can lead to the onset of Type-2 diabetes. It is highly likely that the decreased insulin secretion is mainly defined genetically, and the insulin resistance is considered to be largely attributable to obesity caused by environmental factors such as overeating, high-fat foods, lack of exercise and the like, in addition to genetic factors. In some patients with excess body fat, compensatory hyperinsulinemia reduces the expression of the membrane insulin receptor (IR) leading to insulin resistance as the lack of receptors results in a lowered insulin response. In continuation of these findings, recent report have shown, that defects in processes within the cell itself, such as defects in the insulin signaling pathway, plays a large role in the development of insulin resistance.

Traditional treatment evolves around an increased secretion of insulin from the β-cells, which is known to give side effects, such as e.g., hypoglycemia and weight gain. These side effects are unfortunately seen in both sulfonylureas, acting primarily by stimulating the sulfonylurea-receptor on the β-cells via closure of the K⁺_{ATP}-sensitive channels, and non-sulfonylurea drugs, developed to augment insulin secretion through mechanism other than blocking K⁺_{ATP}-channels. However, these traditionally drugs have not necessarily any impact on the insulin resistance, as the increased secretion of insulin from the β-cells will not compensate for the insensitivity of the response to the secreted insulin.

Preventive actions are urgently needed, and lifestyle changes such as weight control, diet intervention and exercise are first steps. A multifactorial approach, including optimizing weight, blood glucose, blood pressure and lipids, is a most effective tool in preventing diabetic complication. Therapeutic agents with diversified actions, e.g., combined antihyperglycaemic and for example a plasma triglyceride and HDL cholesterol effect, an effect on body weight and/or a blood pressure lowering effect are, therefore, in demand.

Therefore, new pharmacological agents to prevent and/or reduce insulin resistance and treat diseases associated with insulin resistance are needed.

### Summary of invention

The present invention relates to the use of a substance with the core structure of formula (I), or pharmaceutically acceptable salts, solvates or prodrugs thereof, for the manufacture of a composition for the treatment of insulin resistance or diseases associated with insulin resistance, preferably in human subject in a daily dosage in a range of from about 5 mg to about 1500 mg. The invention furthermore relates to a method of treating insulin resistance or diseases associated with insulin resistance in a mammal, said method comprises administering to said mamma, preferably a human subject, a substance with the core structure of formula (I), preferably in a daily dosage in a range from about 5 mg to about 1500 mg. The substance is preferably isosteviol or steviol, or pharmaceutically acceptable salts, solvates or prodrugs thereof. Examples of diseases associated with insulin resistance are e.g. type 2 diabetes mellitus, insulin resistance syndrome, impaired glucose tolerance, the metabolic syndrome, hyperglycemia, hyperinsulinemia, arteriosclerosis, hypercholesterolemia, hypertriglyceridemia, hyperlipidemia, dyslipidemia, obesity, central obesity, polycystic ovarian syndrome, hypercoagulability, hypertension, microalbuminuria, or any combinations thereof.

### Description of Drawings

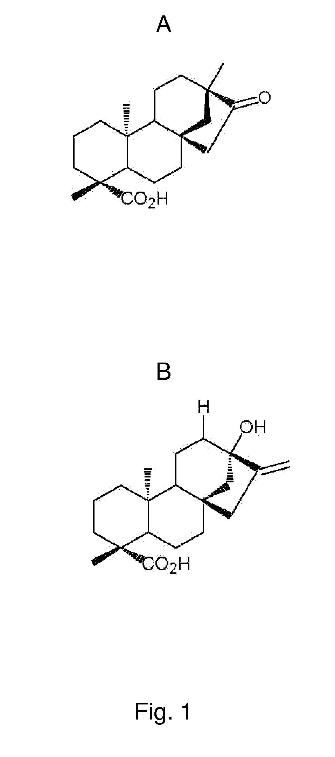

Fig. 1A and 1B shows the structure of isosteviol and steviol, respectively.

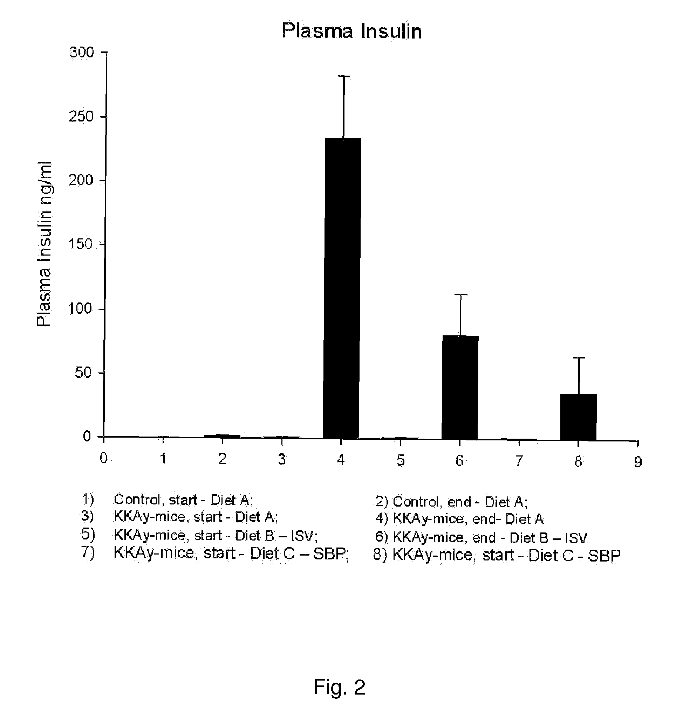

Fig. 2 shows the plasma insulin concentration in normal C57 mice (C57/BL = Control) (n=20) and diabetic KKAy-mice (n=20), before and after a nine weeks treatment period with isosteviol (ISV) (n=10) and Soybean Protein (SBP) (n=10). Data are shown as mean ±SEM.

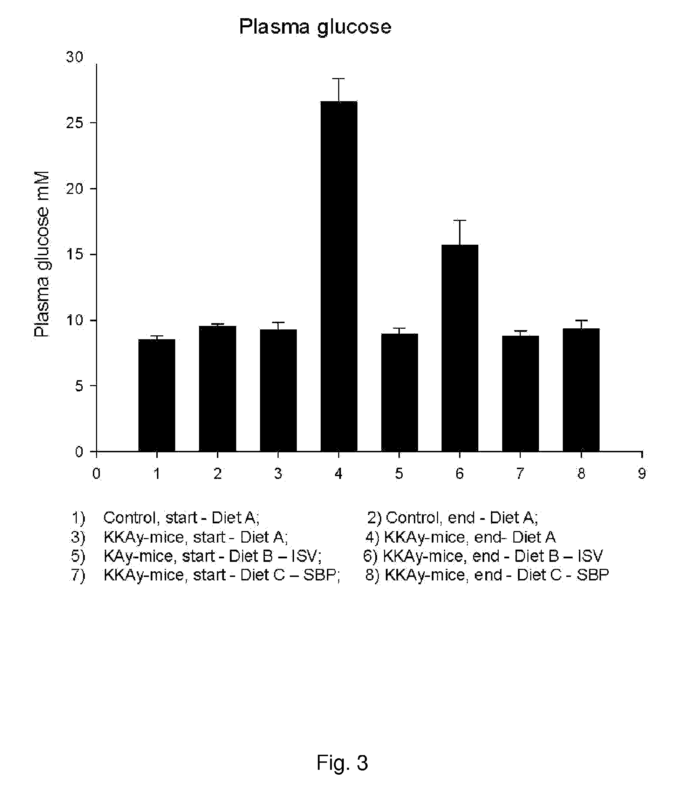

Fig. 3 shows the plasma glucose concentrations in normal C57 mice (C57/BL = Control) (n=20) and diabetic KKAy-mice (n=20), before and after a nine weeks treatment period with isosteviol (ISV) (n=10) and Soybean Protein (SBP) (n=10).

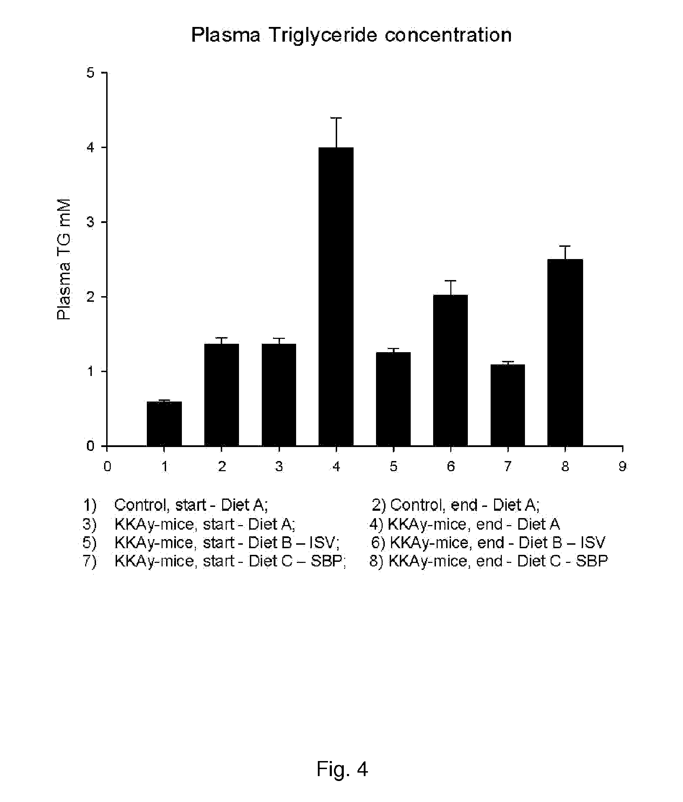

Fig. 4 shows the plasma triglyceride concentration in normal C57 mice (C57/BL=Control) (n=20) and diabetic KKAy-mice (n=20), before and after a nine weeks treatment period with isosteviol (ISV) (n=10) and Soybean Protein (SBP) (n=10). Data are shown as mean ±SEM.

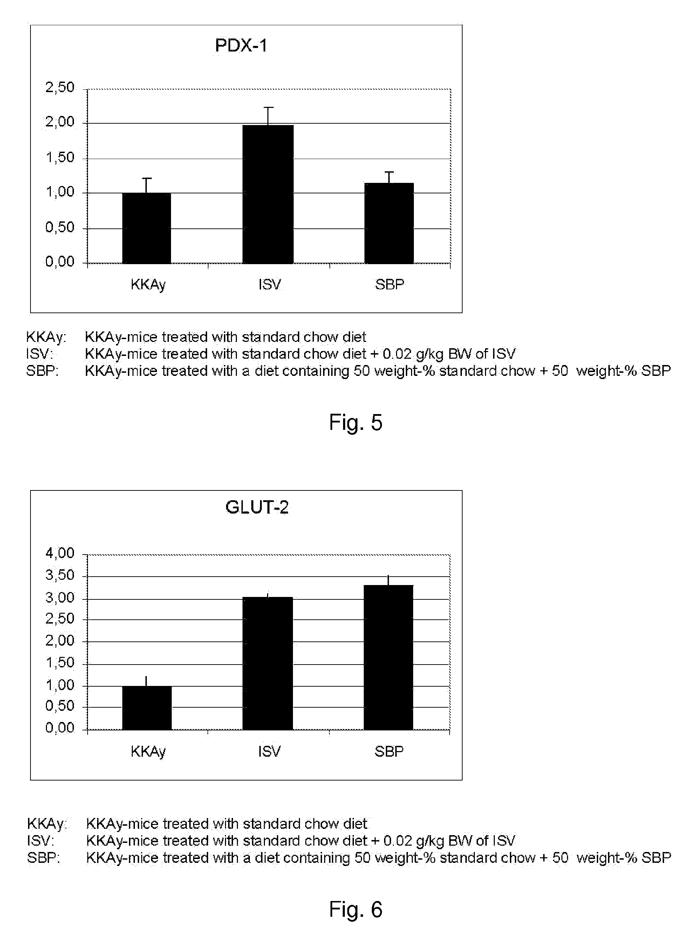

Fig. 5 shows the gene expression profile of the PDX-1 gene in islets from diabetic KKAy-mice, after a nine weeks treatment period with isosteviol (ISV) and Soybean Protein (SBP). Measurements were carried out in triplicate for each sample, and gene expressions were normalized to 18S rRNA expression. Changes in transcript abundance were calculated for the isosteviol group compared to the non-treated control group, *p<0.05 (n=4 in each group). Data are shown as mean ±SEM.
Fig. 6 shows the gene expression profile of the GLUT-2 gene in islets from diabetic KKAy-mice, after a nine weeks treatment period with isosteviol (ISV) and Soybean Protein (SBP). Measurements were carried out in triplicate for each sample, and gene expressions were normalized to 18S rRNA expression. Changes in transcript abundance were calculated for the isosteviol group compared to the non-treated control group, *p<0.05 (n=4 in each group). Data are shown as mean ±SEM.

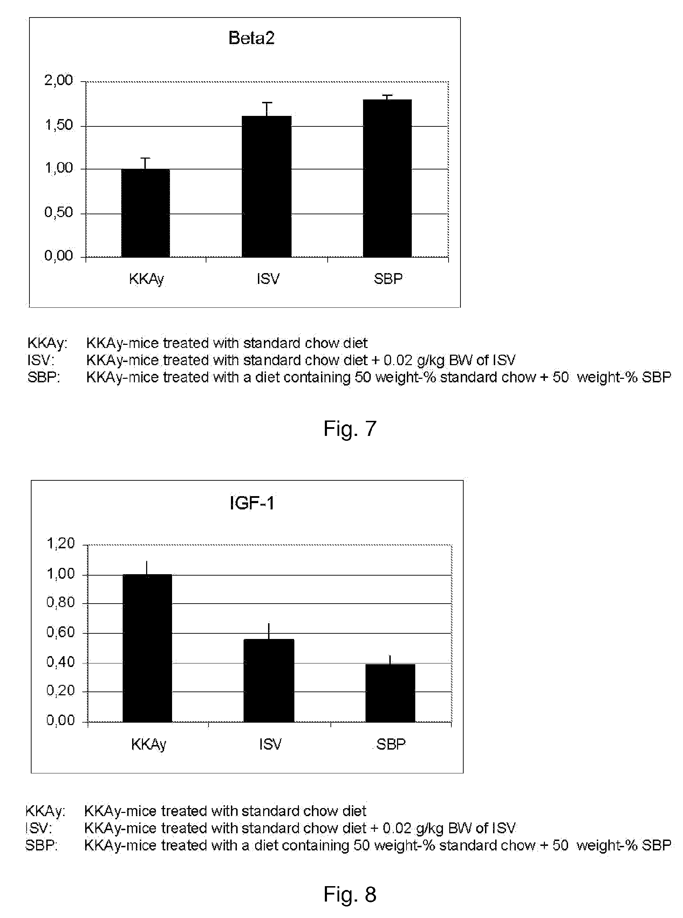

Fig. 7 shows the gene expression profile of the Beta2 gene in islets from diabetic KKAy-mice, after a nine weeks treatment period with isosteviol (ISV) and Soybean Protein (SBP). Measurements were carried out in triplicate for each sample, and gene expressions were normalized to 18S rRNA expression. Changes in transcript abundance were calculated for the isosteviol group compared to the non-treated control group, *p<0.05 (n=4 in each group). Data are shown as mean ±SEM.
Fig. 8 shows the gene expression profile of the IGF-1 gene in islets from diabetic KKAy-mice, after a nine weeks treatment period with isosteviol (ISV) and Soybean Protein (SBP). Measurements were carried out in triplicate for each sample, and gene expressions were normalized to 18S *r*RNA expression. Changes in transcript abundance were calculated for the isosteviol group compared to the non-treated control group, *p<0.05 (n=4 in each group). Data are shown as mean ±SEM.

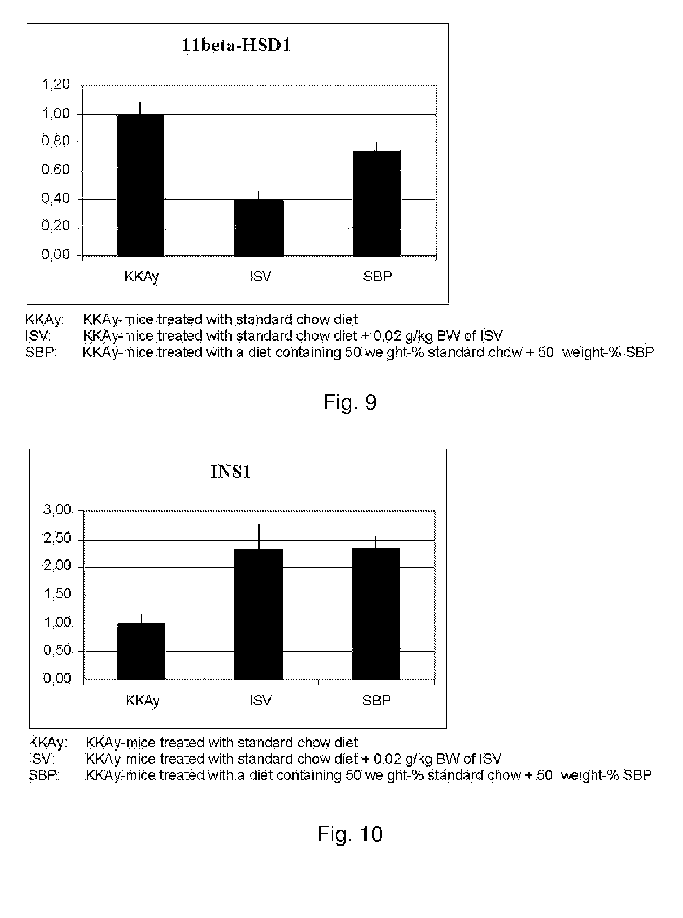

Fig. 9 shows the gene expression profile of the 11 beta-HSD1 gene in islets from diabetic KKAy-mice, after a nine weeks treatment period with isosteviol (ISV) and Soybean Protein (SBP). Measurements were carried out in triplicate for each sample, and gene expressions were normalized to 18S rRNA expression. Changes in transcript abundance were calculated for the isosteviol group compared to the non-treated control group, *p<0.05 (n=4 in each group). Data are shown as mean ±SEM.
Fig. 10 shows the gene expression profile of the INS1 gene in islets from diabetic KKAy-mice, after a nine weeks treatment period with isosteviol (ISV) and Soybean Protein (SBP). Measurements were carried out in triplicate for each sample, and gene expressions were normalized to 18S rRNA expression. Changes in transcript abundance were calculated for the isosteviol group compared to the non-treated control group, *p<0.05 (n=4 in each group). Data are shown as mean ±SEM.

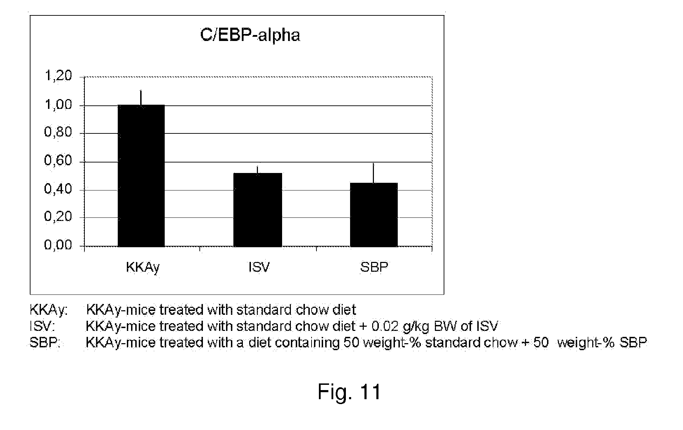

Fig. 11 shows the gene expression profile of the C/EBP-alpha gene in islets from diabetic KKAy-mice, after a nine weeks treatment period with isosteviol (ISV) and Soybean Protein (SBP). Measurements were carried out in triplicate for each sample, and gene expressions were normalized to 18S rRNA expression. Changes in transcript abundance were calculated for the isosteviol group compared to the non-treated control group, *p<0.05 (n=4 in each group). Data are shown as mean ±SEM.

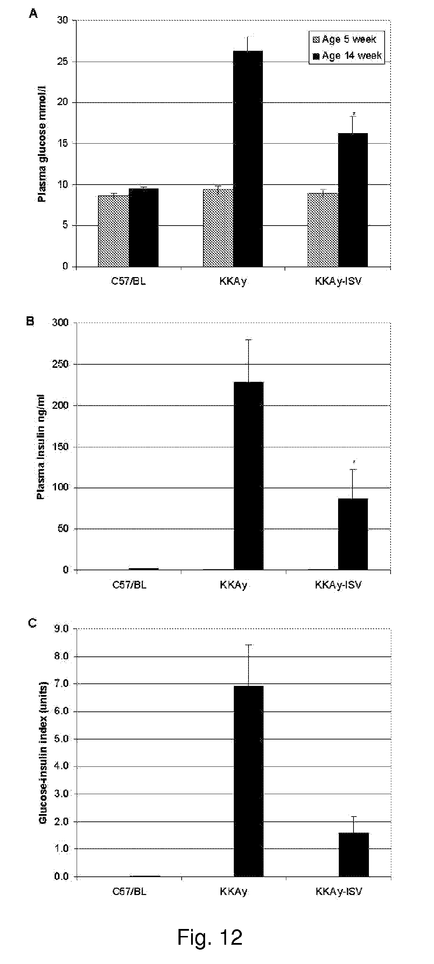

Fig. 12 shows the effect of isosteviol (KKAy-ISV) on fasting plasma glucose (A) and insulin (B), and the glucose-insulin index (C) in KKAy-mice, before and after a nine weeks treatment period. Data are shown as mean ±SEM (n=10 in each group).

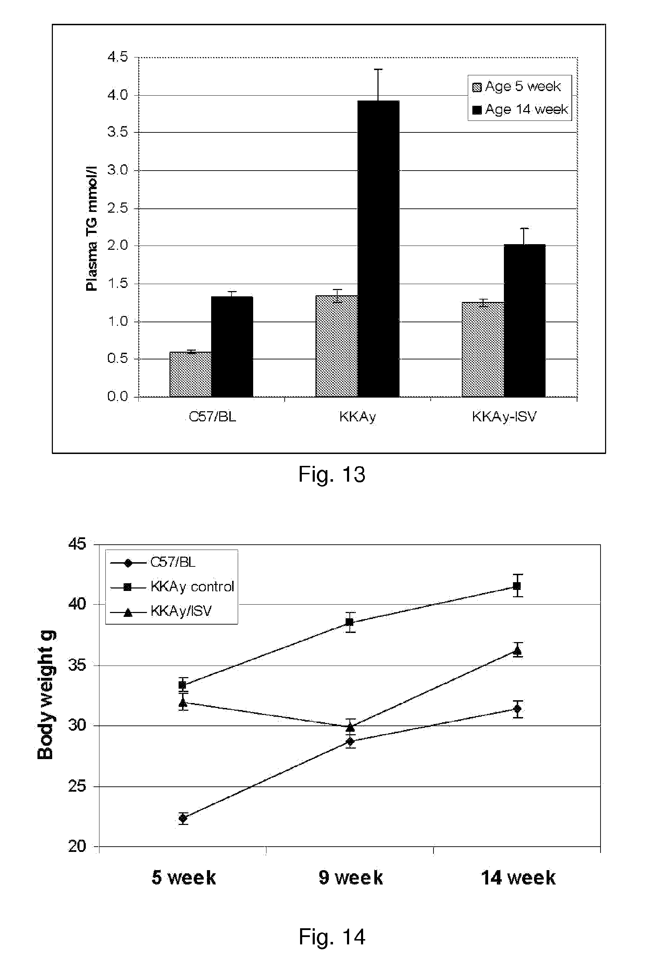

Fig. 13 compares the change in plasma triglyceride in normal C57 mice (C57/BL=Control) and KKAy mice at week 5 and 14. The right columns illustrate the effect of isosteviol on plasma triglyceride levels in KKAy-mice (KKAy-ISV), before and after a nine weeks treatment period. Data are shown as mean ±SEM (n=10 in each group).
Fig. 14 compares the change in body weight in normal C57 mice (C57/BL=Control) and KKAy mice at week 5, 9 and 14. The right columns illustrate the effect of isosteviol on body weight of KKAy-mice (KKAy/ISV). Data are shown as mean ±SEM (n=10 in each group).

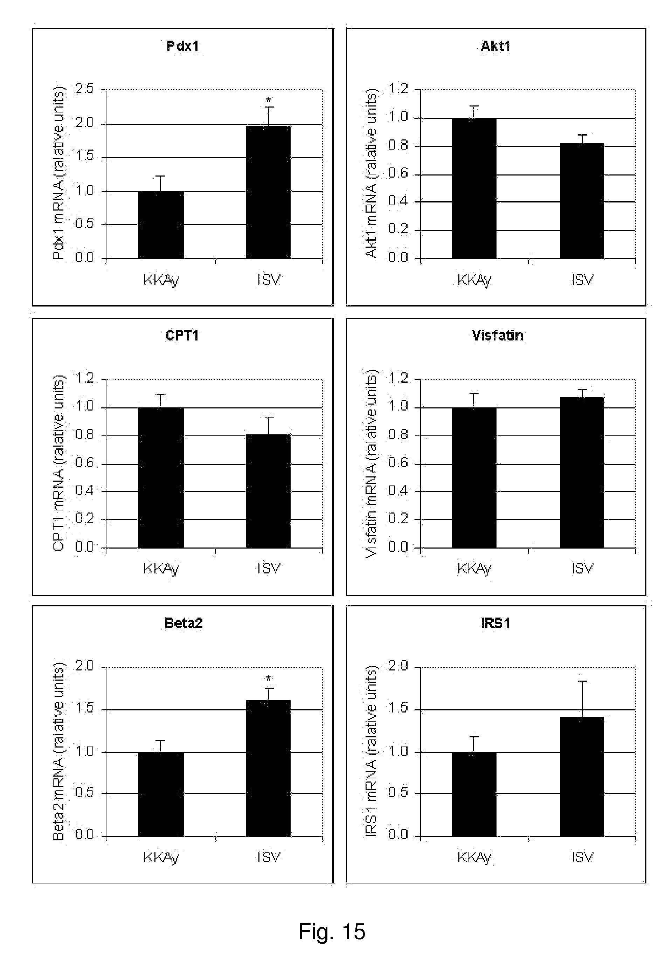

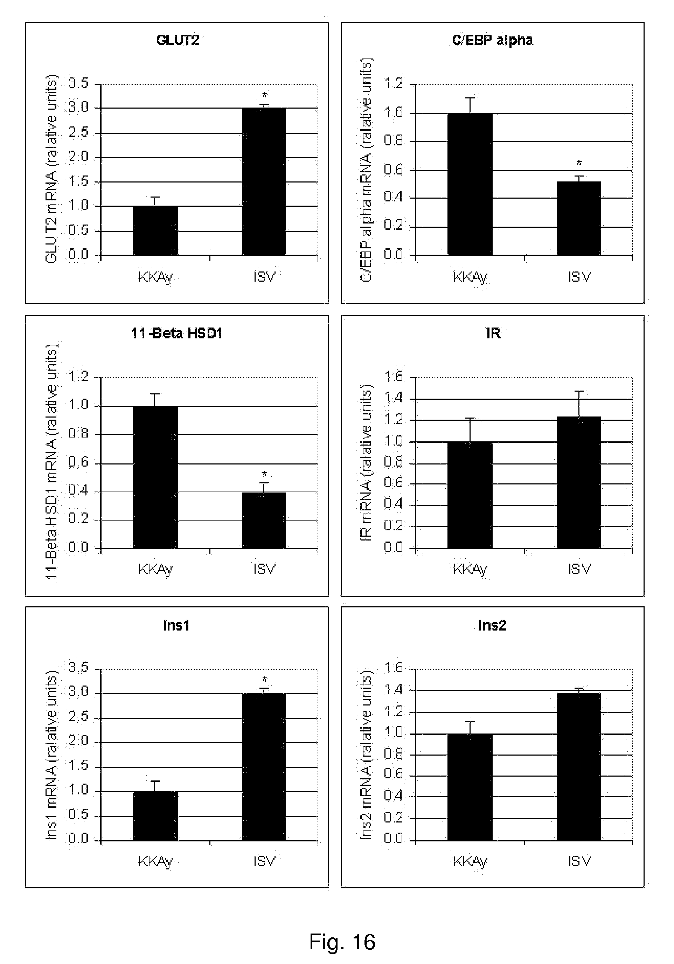

Fig. 15 and 16 shows the results in KKAy mice of changes in mRNA for 12 genes from pancreatic islets treated with isosteviol for 9 weeks (ISV) Measurements were carried out in triplicate for each sample, and gene expressions were normalized to 18S rRNA expression. Changes in transcript abundance were calculated for the isosteviol group compared to the non-treated control group, *p<0.05 (n=4 in each group).

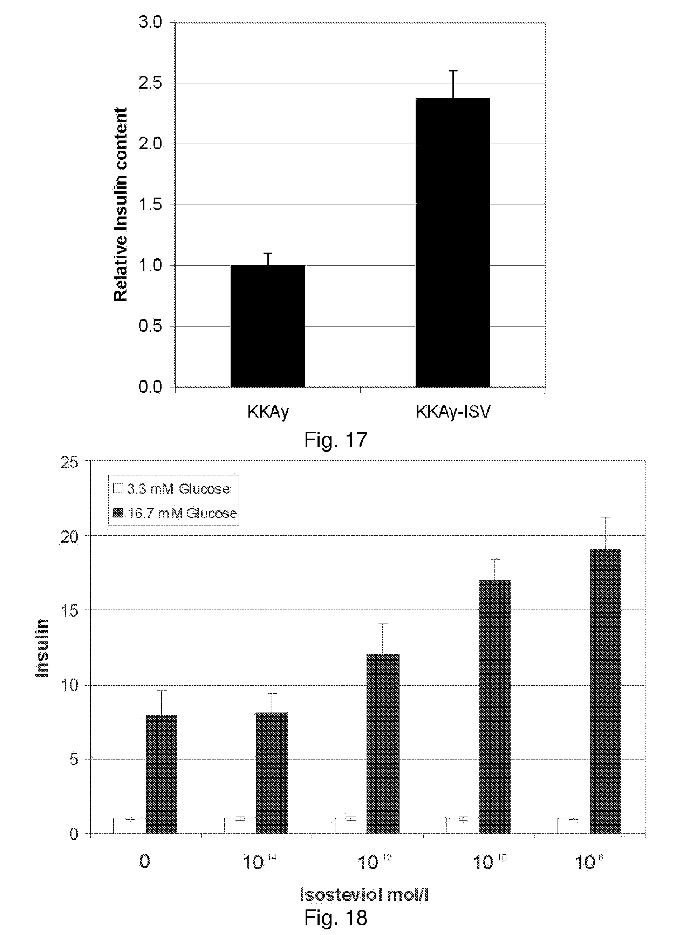

Fig. 17 shows the effect on total insulin protein content in KKAy mice after 9 weeks treatment with isosteviol (KKAy-ISV). Each bar represents mean ± SEM from 3 protein purifications, each pooled from 3-4 mice (n=3 in each bar), *p<0.005 vs. control.
Fig. 18 shows the dose response effect of isosteviol (10⁻¹⁴ mol/l - 10⁻⁸ mol/l) at high (16.7 mmol/l) and low glucose concentrations (3.3 mmol/l) on insulin release from isolated NMRI mice islets. Glucose Stimulated Insulin Secretion (GSIS) for each ISV concentration was measured after 60 min incubation. All measurements at low glucose were set equal to one and the readout at 16.7 mmol/I was adjusted accordingly. Each bar represents the mean ± SEM from 24 single islet incubations, *p< 0.05.

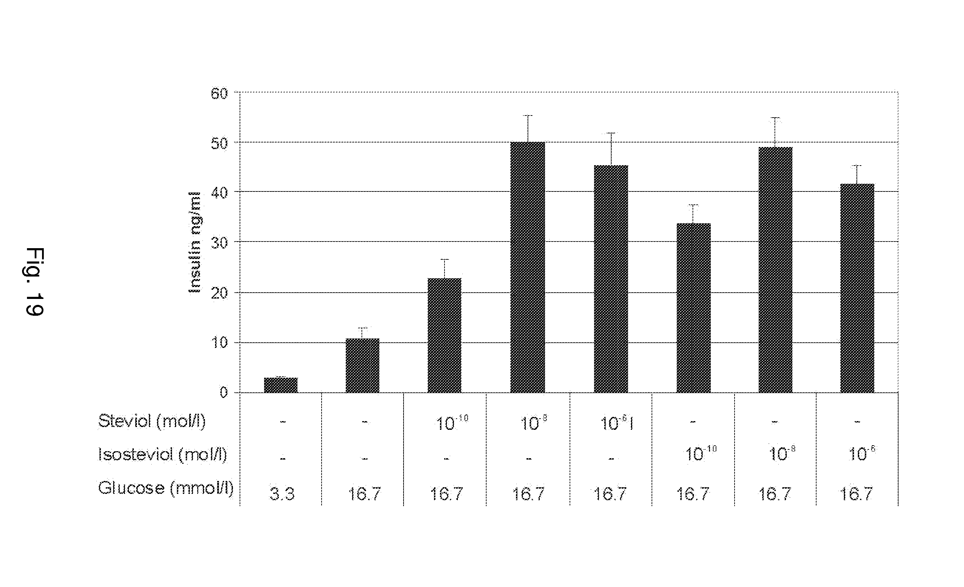

Fig. 19 shows the effects of steviol and isosteviol (10⁻¹⁰ mol/l - 10⁻⁶ mol/l) on glucose (16.7 mmol/l) stimulated insulin secretion (GSIS) from isolated NMRI mice islets incubated 60 minutes in Krebs-Ringer buffer with the indicated concentrations of glucose, isosteviol and steviol. Each bar represents the mean ± SEM from 24 single islet incubations, *p<0.05. The figure shows that steviol and isosteviol stimulate GSIS to the same extent at high concentrations, whereas at 10⁻¹⁰ mol/I isosteviol is more potent than steviol.

### Detailed description of the invention

The present inventors have surprisingly found that substances with the core structure of formula (I), when given to human subjects, can be used in the treatment and prophylaxis of insulin resistance or diseases associated with insulin resistance.

Accordingly, the present invention relates to the use of substances with the core structure of formula (I) 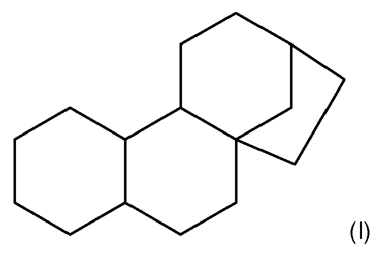 wherein the core structure is substituted with one or more substituents at any chemically feasible positions, or pharmaceutically acceptable salts, solvates or prodrugs thereof, for the manufacture of a composition for the treatment of insulin resistance or diseases associated with insulin resistance in a human subject, wherein the substance is given in a daily dosage in a range of from about 5 mg to about 1500 mg.

In a preferred embodiment of the invention the core structure of formula (I), is a core structure of formula (II) 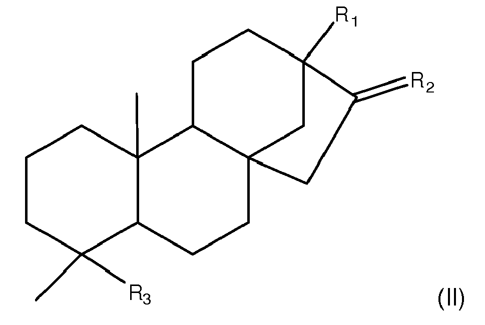 wherein
R₁ is selected from the group consisting of -C₁₋₆alkyl, -O-C₁₋₆alkyl, -OH, and -OC(O)(C₁-₆alkyl), -COO(C₁₋₆alkyl);
R₂ is selected from the group consisting of CH₂, O, and CH(C₁₋₈alkyl);
R₃ is selected from the group consisting of -COOH, -COO(C₁₋₆alkyl), -C(O)NH(C₁₋₆alkyl), -C(O)-(common amino acid moiety); and
wherein the core structure optionally is further substituted with one or more substituents at any chemically feasible positions.

The term "C₁₋₆alkyl" means a saturated linear or branched hydrocarbon group including, for example, methyl, ethyl, isopropyl, t-butyl, pentyl, hexyl, and the like.

The term "common amino acid moiety" means the naturally occurring α-amino acids, unnatural amino acids, substituted β and γ amino acids and their enantiomers. Non-limiting examples are alanine, β-alanine, arginine, asparagine, aspartic acid, cysteine, glutamic acid, glutamine, glycine, histidine, isoleucine, leucine, lysine, methionine, phenylalanine, praline, serine, threonine, tryptophan, tyrosine, valine, 3-hydroxyproline, N-methylphenylalanine, N-methylisoleucine, norvaline, norleucine, ornithine, 2-aminobutyric acid, 2-aminoadipic acid, methionine sulfoxide, methionine sulfone, phenylglycine, o-methyltyrosine, etc.

As is well understood in this technical area, a large degree of substitution is not only tolerated, but is often advisable. Substitution is anticipated on the core structure of substances to be used in the present invention. The term "substituents" are used to differentiate between the core structure of formula (I) and formula (II) and further chemical species that may be substituted on to the core structure. Non-limiting examples of suitable substituents may be hydrocarbon alkyl substituents, such as methyl, ethyl, propyl, t-butyl, and the like, and further substituents known in the art, such as hydroxy, alkoxy, alkylsulfonyl, halogen, cyano, nitro, amino, carboxyl, aryl, heteroaryl, cycloalkyl, common amino acids etc. It is well-known that these substituents may include further substitution, such for example, alkyl, aryl, heteroaryl etc. bearing further substituents known in the art, such as hydroxy, alkoxy, alkylsulfonyl, halogen atoms, cyano, nitro, amino, carboxyl, common amini acids etc.

The term "aryl" means a mono- or polycyclic aromatic hydrocarbon group.

The term "heteroaryl" means a monovalent aromatic cyclic radical having one to three rings, of four to eight atoms per ring, incorporating one or two heteroatoms (chosen from nitrogen, oxygen, or sulphur) within the ring.

The term "cycloalkyl" means a monovalent saturated carbocyclic radical consisting of one, two or three rings, of three to eight carbons per ring.

When the substances of the present invention contain asymmetric carbon atoms, the pharmaceutical acceptable salts, solvates and prodrugs may exist as single stereoisomers, racemates, and/or mixtures of enantiomers and/or diastereomers. All such single stereoisomers, racemates, and mixtures thereof are intended to be within the scope of the present invention.

An even more preferred embodiment of the present invention relates to the use of a substance selected from the group consisting of isosteviol and steviol, or pharmaceutically acceptable salts, solvates or prodrugs thereof, for the manufacture of a composition for the treatment of insulin resistance or diseases associated with insulin resistance in a human subject, wherein the substance is given in a daily dosage in a range of from about 5 mg to about 1500 mg. In a preferred embodiment, the substance is isosteviol, or pharmaceutically acceptable salts, solvates or prodrugs thereof. Alternatively, the substance is steviol, or pharmaceutically acceptable salts, solvates or prodrugs thereof. However, in some embodiments the substance may furthermore be a mixture of steviol and isosteviol, or pharmaceutically acceptable salts, solvates or prodrugs thereof. The structure of isosteviol (ent-16-ketobeyeran-19-oic acid) and steviol (ent-kaur-16-en-13-ol-19-oic acid) can be seen in figure 1A and 1B, respectively.

The term "pharmaceutically acceptable" means that the substance or composition must be compatible with the other ingredients of a formulation, and not deleterious to the patient.

The terms "treating", "treat" or "treatment" include both preventative (e.g., prophylactic), palliative, and curative treatment, together with a treatment to reduce symptoms.

The present inventors have surprisingly found that the effects of the substances to be used in accordance with the invention can be used in both an insulin sensitivity adjusting treatment, a glucose sensitivity adjusting treatment or, where necessary a treatment combining an insulin and glucose sensitivity adjusting treatment.

In a preferred embodiment of the invention the composition is for the treatment of insulin resistance, and in another embodiment the composition is for the treatment of diseases associated with insulin resistance. The term "diseases associated with insulin resistance" means any disease, condition, or disorder, wherein any more or less progressed insulin resistance plays a role. Non limiting examples of diseases and/or conditions associated with insulin resistance may be selected from the group consisting of Type 2 diabetes mellitus, insulin resistance syndrome, impaired glucose tolerance, the metabolic syndrome, hyperglycemia, hyperinsulinemia, arteriosclerosis, hypercholesterolemia, hypertriglyceridemia, hyperlipidemia, dyslipidemia, obesity, central obesity, polycystic ovarian syndrome, hypercoagulability, hypertension, microalbuminuria, and any combinations thereof.

In one embodiment of the invention, diseases associated with insulin resistance is preferably selected from the group consisting of Type 2 diabetes mellitus, insulin resistance syndrome (IRS), impaired glucose tolerance, the metabolic syndrome, hyperglycemia, and hyperinsulinemia. In another embodiment of the invention the disease associated with insulin resistance is preferably arteriosclerosis.

Alternatively, in a further embodiment of the invention, diseases associated with insulin resistance is preferably selected from the group consisting of hypercholesterolemia, hypertriglyceridemia, hyperlipidemia, dyslipidemia, hypertension, microalbuminuria, hypercoagulability, polycystic ovarian syndrome, obesity, central obesity and combinations thereof.

When the substances as defined by formula (I), e.g., steviol or isosteviol, or their pharmaceutically acceptable salts, solvates or prodrugs, are given to human subjects in accordance with the present invention, for treatment and/or prophylaxis of insulin resistance or diseases associated with insulin resistance, then an improved glycemic control is seen. This improved glycemic control is reflected in for example various diagnostic values, such as for example fasting and post prandial plasma glucose levels and HbA1c. The overall therapeutic effect may furthermore be seen and measured as one or more of the following, non-limiting values: lower plasma glucose concentration, lower triglyceride levels, a reduced blood pressure, a reduced body weight, and an improvement in the coagulation state. Accordingly, the diversified actions of substances as defined by formula (I), e.g., steviol or isosteviol, or any pharmaceutically acceptable salts, solvates or prodrugs thereof, are not only influencing the glycemic level but also the underlying insulin resistance, hereby influencing the entire range of accompanying factors and lowering the overall risk of for example cardiovascular diseases, the metabolic syndrome, i.e., the insulin resistance syndrome, and development of Type-2 diabetes mellitus. The effect on insulin resistance is further illustrated in the clinical study described in the experimental section, example 6.

As used herein the term "HbA1c" means the widely used expression for the amount of glycosylated hemoglobin in blood, expressed in %. HbA1 c gives a measure of the long-term serum glucose regulation, as the HbA1 c level is proportional to the average blood glucose concentration over the previous four weeks to three months. The normal range found in healthy subjects is about 4% to about 5.9%. Higher levels represent poor glycemic control and subjects with diabetic conditions may often have higher levels of HbA1 c. Accordingly, the HbA1 c gives a measure of how well diabetic conditions are being managed, and a reduction of the HbA1 c value after initiation of a treatment may therefore be interpreted as an improved glycemic control due to the treatment. Treatment with the substances according to the present invention, e.g., steviol or isosteviol, or pharmaceutically acceptable salts, solvates or prodrugs will therefore give a decrease in HbA1 c, such as e.g. a 0,25% decrease, a 0,25 % decrease, or a 0,50% decrease, and preferably a decrease in HbA1 c to the normal range found in healthy subjects, i.e., from about 4% to about 5.9%.

As used herein the term "post prandial" means after a meal, and is especially used in connection with the blood glucose level measured after a meal.

As used herein, the term "salt" includes, but is not limited to, any possible base or acid addition salts of the substances steviol and isosteviol. The acid addition salts are formed from basic substances, whereas the base addition salts are formed from acidic substances. All of these forms are within the scope of the present invention. A non-toxic pharmaceutically acceptable base addition salt of an acidic substance may be prepared by contacting the free acid form of the substance with a sufficient amount of a desired base to produce the salt in the conventional manner. The free acid form of the substance may be regenerated by contacting the salt form so formed with an acid, and isolating the free acid of the substance in the conventional manner. The free acid forms of the substances differ from their respective salt forms somewhat in certain physical properties such as solubility, crystal structure, hygroscopicity, and the like, but otherwise the salts are equivalent to their respective free acid for purposes of the present invention. Non limiting examples of counter ions for the base additions salts are a metal cation, such as an alkali or alkaline earth metal cation, or an amine, especially an organic amine. Examples of suitable metal cations include sodium cation (Na+), potassium cation (K+), magnesium cation (Mg2+), calcium cation (Ca2+), and the like. Examples of suitable amines are N,N'-dibenzylethylenediamine, chloroprocaine, choline, diethanolamine, dicyclohexylamine, ethylenediamine, N-methylglucamine, and procaine (see, for example, Berge S.M. et al., "Pharmaceutical Salts," J. of Pharma. Sci., 1977;66:1).

As used herein, the term "solvate" means a substance of the invention or a salt thereof that further includes a stoichiometric or non-stoichiometric amount of a solvent bound by non-covalent intermolecular forces. Preferred solvents are volatile, non-toxic, and/or acceptable for administration to humans in trace amounts. The solvated forms, including hydrated forms, are equivalent to unsolvated forms and are encompassed within the scope of the present invention.

As used herein, the term "prodrug" means a substance that is transformed *in vivo* to yield a substance of the present invention. The transformation may occur by various mechanisms, such as through hydrolysis in blood. For example, when a compound of the present invention contains a carboxylic acid functional group, a prodrug can comprise an ester formed by the replacement of the hydrogen atom of the acid group with a group including, but not limited to, groups such as for example (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N (alkoxycarbonyl)amino)ethyl having from 4 to 10 carbon atoms, 3-phthalidyl, 4 crotonolactonyl, gamma-butyrolacton-4-yl, di-N,N-(C₁-C₂)alkylamino(C₂-C₃)alkyl, carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl. The a prodrug can furthermore comprise e.g. an amide formed by the replacement of the hydrogen atom of an acid group with a common amino acid moiety, non-limiting examples of common amino acids are mentioned herein above.

In the context of the present invention, the term "daily dosage" is meant to describe the daily dosage required for an average human subject having a weight of about 65 to about 70 kg. In general, for administration to human patients the daily dosage level of the substances for use in accordance with the present invention, is in a range of from about 5 mg to about 1500 mg.

In one embodiment of the present invention the substance is given in a daily dosage in a range of from about 5 mg to about 500 mg, such as e.g., from about 10 mg to about 500 mg, about 20 mg to about 500 mg, about 30 mg to about 500 mg, about 40 mg to about 500 mg, about 50 mg to about 500 mg, about 100 mg to about 500 mg, about 5 mg to about 400 mg, about 5 mg to about 300 mg, about 5 mg to about 200 mg, about 5 mg to about 100 mg, about 100 mg to about 500 mg, about 100 mg to about 400 mg, about 100 mg to about 300 mg, about 100 mg to about 200 mg, about 200 mg to about 500 mg, about 200 mg to about 400 mg, or about 200 mg to about 300 mg.

In another embodiment of the present invention the substance is given in a daily dosage in a range of from about 500 mg to about 1000 mg, such as e.g., from about 500 mg to about 900 mg, about 500 mg to about 800 mg, about 500 mg to about 700 mg, about 500 mg to about 600 mg, about 600 mg to about 1000 mg, about 700 mg to about 1000 mg, about 800 mg to about 1000 mg, about 900 mg to about 1000 mg, or about 600 mg to about 900 mg.

In a further embodiment of the present invention the substance is given in a daily dosage in a range of from about 1000 mg to about 1500 mg, such as e.g., from about 1000 mg to about 1400 mg, about 1000 mg to about 1300 mg, about 1000 mg to about 1200 mg, about 1000 mg to about 1100 mg, about 1100 mg to about 1500 mg, about 1200 mg to about 1500 mg, about 1300 mg to about 1500 mg, about 1400 mg to about 1500 mg, or about 1100 mg to about 1400 mg.

According to the inventors, the therapeutic effect seen from the administration of steviol in connection with treatment of diseases associated with insulin resistance actually arise from isosteviol, resulting from steviol undergoing a rearrangement to isosteviol in the acidic environment of the stomach. An advantage of isosteviol over steviol is therefore the presence of 100 % active compound. Accordingly, in a preferred embodiment of the present invention the substance used is isosteviol, or pharmaceutically acceptable salts, solvates or prodrugs thereof. If for instance only a 60 or 70% rearrangement of steviol to isosteviol takes place in the acidic environment of the stomach, then this would account for the lesser *in vivo* effect seen from steviol than from isosteviol. Accordingly, the daily dose required to give a desired therapeutic effect is higher for steviol than for isosteviol.

In a preferred embodiment of the present invention the substance is isosteviol, or pharmaceutical acceptable salts, solvates or prodrugs thereof, and the daily dosage is in a range of from about 100 mg to about 1000 mg, preferably from about 500 mg to about 1000 mg. Alternatively, the substance is steviol, or pharmaceutical acceptable salts, solvates or prodrugs thereof, and the daily dosage is in a range of from about 100 mg to about 1000 mg, preferably from about 500 mg to about 1000 mg.

The skilled person will readily be able to determine the dosage levels required for a subject whose weight falls outside the average range, such as children and the elderly. The daily dosage may optionally be administered as a single dose or be divided in two or more doses, such as e.g. two, three, or four, for administration at different times during the day. The skilled person will appreciate that, in the treatment of insulin resistance or diseases associated with insulin resistance, substance used in accordance with the presents invention may be taken as a single dose on an "as required" basis, i.e., as needed. The physician will in any event determine the actual dosage which will be most suitable for any particular patient and it will vary with the age, weight and response of the particular patient. The above dosages are, of course only exemplary of the average case and there may be instances where higher or lower doses are merited and such are within the scope of the invention.

Another way of expressing the daily dosage level in accordance with the present invention is as mg/kg. Accordingly, for administration to human patients the daily dosage levels of the substances in accordance with the present invention, or pharmaceutically acceptable salts, solvates or prodrugs thereof, will be in a range from about 0.06 to about 20 mg/kg, preferably from about 1,5 to about 14 mg/kg, and more preferably from about 7 to about 14 mg/kg.

The phrase "pharmaceutically acceptable salt(s)," as used herein, includes but are not limited to salts of acidic groups that may be present in steviol or isosteviol. Acidic groups, such as e.g., carboxylic acids, are capable of forming base salts with various pharmacologically acceptable cations. Examples of such salts include alkali metal or alkaline earth metal salts and, particularly, calcium, magnesium, sodium lithium, zinc, potassium, and iron salts.

The substances for use in accordance with the present invention may be administered alone, or as part of a combination therapy. If a combination of active agents is administered, then it may be administered simultaneously, separately or sequentially. Depending on the disease and the state of the disease to be treated, it may be relevant to include one or more additional active substance in the medicament. In particular, in one embodiment of the present invention, the substances for use in connection with the treatment of insulin resistance or diseases associated with insulin resistance is combined with one or more additional active substances selected from the group consisting of insulin, sulfonylureas, meglitinides, biguanides, thiazolidinediones, glitazones, α-glucosidase inhibitors, incretin mimetics such as e.g. GLP-1 analogues and GLP-1 agonists, DPP-4 inhibitors, amylin analogues, PPAR α/γ ligands, sodium-dependent glucose transporter 1 inhibitors, fructose 1,6-bisphosphatase inhibitors, glucagon inhibitors, and 11 beta-HSD1 inhibitors. Non-limiting examples of the one or more additional active substance may be selected from the group consisting of insulin, glimepiride, glibenclamide, tolbutamide, gliclazide, glipzid, repaglinide, nateglinide, metformin, pioglitazones, rosiglitazones, acarbose, miglitol, liraglutide, exenatide, sitagliptin, vildagliptin saxagliptin, and alogliptin.

In another embodiment of the present invention the one or more additional active substances are selected from the group consisting of thiazides, diuretics, ACE inhibitors, AT2 inhibitors, ARB, Ca²⁺ antagonists, α-blockers, β-blockers, cholesterol absorption inhibitors, hypolipidemic drugs, fibrates, anion exchangers, bile acid sequestrants, fish oils, HMG-CoA reductase inhibitors, and CB1 cannabinoid receptor antagonists. Non-limiting examples of the one or more additional active substance may be selected from the group consisting of bendroflumetiazid, indapamid, hydrochlorothiazid, captopril, enalapril, lisinopril, fosinopril, perindopril, quinapril, ramipril, trandolapril, quinapril, fosinopril, candesartancilexetil, irbesartan, losartan, valsartan, telmisartan, eprosartan, olmesartanmedoxomil, nifedipin, amlodipin, nitrendipin, diltiazem, felodipin, verapamil, lacidipin, isradipin, lercanidipin, doxazosin, prazosin, terazosin, phentolamin, hydralazin, acebutolol, atenolol, bisoprolol, carvedilol, esmolol, labetalol, metoprolol, pindolol, propranolo, sotalol, tertatolol, timolol, methyldopa, moxonidin, ezitimibe, gemfibrozil, bezafibrat, fenofibrate, nicotinic acid, acipimox, colestipol, colestyramin, fish oils, atorvastatin, fluvastatin, lovastatin, pravastatin, rosuvastatin, simvastatin, and rimonabant.

In one embodiment of the present invention, the composition is for oral, peroral, sublingual, parenteral, intramuscular, topical, buccal, nasal, or inhalation administration. In a preferred embodiment of the present invention, the medicament is for oral administration.

Another aspect of the present invention relates to the use of a substance with the core structure of formula (I), as defined above, or pharmaceutically acceptable salts, solvates or prodrugs thereof, for the manufacture of a composition for the treatment of insulin resistance. In a preferred embodiment of this aspect of the invention, the core structure of formula (I), is a core structure of formula (II), as defined above. In a more preferred embodiment, the substance is selected from isosteviol and steviol, or pharmaceutically acceptable salts, solvates or prodrugs thereof; and in an even more preferred embodiment, the substance is isosteviol, or pharmaceutically acceptable salts, solvates or prodrugs thereof.

Preferably the insulin resistance is in a mammal, such as e.g., a human subject. The substance, or pharmaceutically acceptable salts, solvates or prodrugs thereof, may for example be given to a human subject in a daily dosage in a range of from about 5 mg to about 1500 mg. Preferably the daily dosage is in a range of from about 100 mg to about 1000 mg, such as more preferably from about 500 mg to about 1000 mg. A composition may further comprise one or more additional active substances. These further active substances, together with applicable dosage ranges is described above for the first aspect of the invention, and apply *mutatis mutandis* together with the other previously described embodiments, for this aspect of the invention.

### Method of treatment

The presents invention furthermore encompasses a method of treating insulin resistance or diseases associated with insulin resistance in a human subject, said method comprises administering to said human subject a substance with the core structure of formula (I) 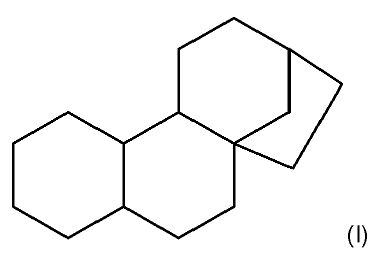 wherein the core structure is substituted with one or more substituents at any chemically feasible positions, or pharmaceutically acceptable salts, solvates or prodrugs thereof, in a daily dosage in a range from about 5 mg to about 1500 mg.

In a preferred embodiment of the method according to the invention, the core structure of formula (I), is a core structure of formula (II) 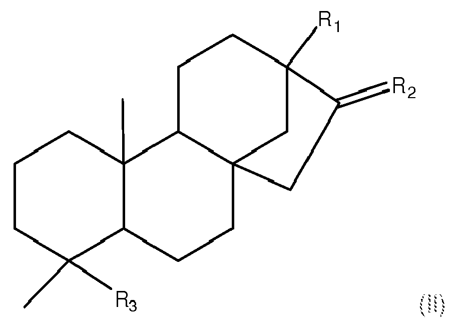 wherein
R₁ is selected from the group consisting of -C₁₋₆alkyl, -O-C₁₋₆alkyl, -OH, and -OC(O)(C₁₋₆alkyl), -COO(C₁₋₆alkyl);
R₂ is selected from the group consisting of CH₂, O, and CH(C₁₋₆alkyl);
R₃ is selected from the group consisting of -COOH, -COO(C₁₋₆alkyl), -C(O)NH(C₁₋₆alkyl), -C(O)-(common amino acid moiety); and
wherein the core structure optionally is further substituted with one or more substituents at any chemically feasible positions.

In an even more preferred embodiment of the method according to the present invention, the substance is selected from the group consisting of steviol or isosteviol, or pharmaceutically acceptable salts, solvates or prodrugs thereof.

In a preferred embodiment the daily dosage is in a range of from about 100 mg to about 1000 mg, preferably from about 500 mg to about 1000 mg.

In another aspect, the present invention relates to a method of treating insulin resistance, said method comprises administering a substance with the core structure of formula (I), as defined above, or pharmaceutically acceptable salts, solvates or prodrugs thereof, to a mammal. In a preferred embodiment of said method, the core structure of formula (I), is a core structure of formula (II), as defined above. In another preferred embodiment of said method, the substance is selected from isosteviol and steviol, or pharmaceutically acceptable salts, solvates or prodrugs thereof. In an even more preferred embodiment, the substance is isosteviol, or pharmaceutically acceptable salts, solvates or prodrugs thereof.

In one embodiment of said method the mammal is a human, and in an even more preferred embodiment of said method the substance is administered to a human in a daily dosage in a range from about 5 mg to about 1500 mg, preferably from about 100 mg to about 1000 mg, and more preferably from about 500 mg to about 1000 mg.

The features mentioned above for the use of a substance, or pharmaceutically acceptable salt, solvates or prodrugs thereof, for the preparation of a medicament, apply *mutatis mutandis* for the methods of treatment according to the present invention.

### In vivo study in mice

The inventors have performed *in vivo* investigations in mice of the effect of the substances in accordance with the invention on the long-term plasma concentrations of insulin, glucose and triglycerides. The obese Type-2 diabetes melitus mice, the KKAy mice, can serve as a useful model for assaying an effect on insulin resistance, or diseases associated with insulin resistance, since these animals progress through several developmental stages to overt diabetes in a relatively predictable age-dependent fashion, when maintained under standard conditions. The KKAy mice rapidly progress from an insulin resistant stage with hyperinsulinaemia and euglycaemia to a hyperglycaemic, insulin-deficient stage at the age of approximately 12 weeks.

By measuring plasma glucose concentration, plasma insulin concentration, plasma triglyceride concentration, and hormone levels in blood samples at start and at end of the treatment period, together with some weekly measurements of fasting blood glucose and body weight, the assay in KKAy mice can be used to show an increase in insulin and glucose sensitivity.

It is especially noteworthy to follow the plasma glucose concentration, as if the plasma glucose concentration decline concomitantly with a lower insulin concentration, the anti-hyperglycaemic effect does not arise from an increase in insulin but arise from an increased sensitivity towards the smaller amount of insulin secreted. Furthermore, a lower plasma triglyceride concentration is an indication of increased insulin sensitivity.

Dysfunction of the insulin-producing beta cell of the pancreas during development of insulin resistance is recognized as a major reason for escalation of peripheral insulin resistance and progression to overt Type-2 diabetes mellitus. It is therefore essential to clarify the gene changes in the beta cell during the development of insulin resistance, including changes in genes involved in glucose sensing (GLUT2), transcription factors regulating insulin expression and beta cell function (Pdx1, Beta2, Pax6, Foxa2 Nkx2.2, Nkx6.1) and the insulin signaling pathway (IR, IRS, Akt). Accordingly, by assaying the long-term effect of substances on the gene expression profile of the key insulin regulatory genes involved in the insulin pathway or genes otherwise related to insulin resistance, the effect on insulin resistance or diseases associated with insulin resistance may be further established. From the above-mentioned *in vivo* studies in mice, RNA from Islets can be purified and the gene expression of PDX-1, GLUT2, Beta2, IGF1, 11beta-HSD-1, Ins1, C/EBP-alpha, IRS1, Akt1, CPT1, and IR can be measured with Real time RT-PCR.

The above-mentioned genes are furthermore candidate genes for identifying individuals at risk for the development of insulin resistance or to develop new pharmacological agents. At present there are few reliable methods for presymptomatic diagnosis of a genetic predisposition for e.g. type II diabetes.

Pdx-1 (pancreatic duodenal homeobox gene-1) is a homeodomain transcription factor essential for pancreatic development and mature beta cell function and plays a key role in normal insulin secretion by islets. (Edlund, 1998). The Pdx-1 gene is initially expressed in exocrine and endocrine pancreatic precursors but later becomes restricted mainly to β cells. Loss of Pdx-1 expression leads to pancreatic agenesis and haploinsufficiency of the Pdx-1 gene results in defects in glucose-stimulated insulin secretion in humans and mice (Ahlgren et al., 1998). Some recent studies indicate that mutations in Pdx-1 may predispose individuals to late onset type 2 diabetes due to insulin resistance; (Hansen et al., 2000). Hereby indicating, that a lowering of the Pdx-1 expression may contribute to the development of type II diabetes by causing impaired expression of GLUT-2 and insulin. Accordingly, by stimulating Pdx-1 expression insulin resistance may be prevented, treated or reduces. An assay for Pdx-1 expression may be used to assay for an effect on insulin resistance.

GLUT-2 is a transmembrane protein which is involved in passive transport of glucose over cellular membranes e.g the liver and pancreatic β-cells. The receptor is insulin independent. Glucose-stimulated insulin secretion by the β-cells is a highly regulated process in which GLUT-2 and glucokinase (GK) have been proposed to play important roles. As GLUT-2 is involved in the passive transport of glucose over cellular membranes e.g in the liver and pancreatic β-cells, an increased expression of GLUT-2 will lead to an improved glucose sensitivity and hereby an reduced insulin resistance. An assay for GLUT-2 expression may therefore be used to assay for an effect on insulin resistance.

Beta2/NeuroD is a key regulator of both insulin genes transcription in pancreatic beta-cells in which heterozygous mutations is found associated with the development of Type-2 diabetes mellitus (Habener et al. 2005, Malecki et al. 1999). Resent studies indicate, that impairment of Beta2 expression relates to insulin resistance. Accordingly, an increase in the overall expression of Beta2 implies an effect on insulin resistance, and an assay for Beta2 expression may be used to assay for an effect on insulin resistance.

IGF-1 (Insulin-like growth factor 1) is a polypeptide protein hormone similar in molecular structure to insulin. It plays an important role in childhood growth and continues to have anabolic effects in adults. Usala et al, 1992, found that diabetic patients with extreme insulin resistance have substantial improvement in metabolic control during administration of IGF-1. A reduced expression of IGF-1 may therefore have a potential positive effect on the risk of developing angiogenesis and microvascular complications e.g. reduced risk of diabetic retinopathy. An assay for IGF-1 expression may therefore be used to e.g. assay for an effect on diseases associated with insulin resistance.

11beta-HSD1 (11-Beta Hydroxysteroid Dehydrogenase) is the name of a family of enzymes that catalyzes the conversion of inert 11 keto-products (cortisone) to active cortisol, or vice versa, thus regulating the access of glucocorticoids to the steroid receptors. In rodents, 11-beta-HSD-1 converts 11-dehydrocorticosterone (DHC) into corticosterone and, in humans, it converts cortisone into cortisol. 11 beta-HSD1 is widely expressed, particularly in the liver. Glucocorticoids play a major role in glucose homeostasis by influencing hepatic gluconeogenesis and glycogen degradation. Genetic deletion of 11 beta-HSD1 lowers plasma glucose levels in mice on high-fat diets and attenuates the activation of enzymes involved in hepatic gluconeogenesis suggesting that inhibitors of this enzyme may be of use in various metabolic disorders. Many effects of glucocortioids directly oppose the effects of insulin, thereby inducing insulin resistances. Because of the beneficial effect of a reduced glucocorticoid level, 11-beta-HSD-1 is a desired target for pharmacological intervention. For examble, glucocorticoids impair insulin dependent glucose intake in the peripheral tissue, enhances glucose production in the liver, and inhibits insulin secretion from pancreatic beta-cells. Accordingly, a down regulation of 11beta-HSD-1 will have a positive effect on insulin resistance. An assay for e.g. an decreased expression of 11 beta-HSD-1 may be used to assay an effect on insulin resistance or diseases associated with insulin resistance.

Ins1 is one of the two insulin expressing genes found in mice, if a rise in INS gene expression takes place concomitantly with increased GLUT-2 expression it will indicate an improved glucose tolerance due to improved insulin secretion. Accordingly, an assay for Ins1 expression, together with the above-mentioned GLUT-2 expression, may further give an indication of an improved insulin tolerance.

The C/EBP (CCAAT/enhancer-binding protein) family of transcriptional regulators is critically important for the activation of adipogenic genes during differentiation. Glucose and insulin in excess are capable of down-regulating CCAAT enhancer binding protein α (C/EBPα), a transcription factor essential for maintenance of a fully differentiated adipocyte phenotype (2-4) (Reusch and Klemm; 1999). This is an important observation because loss of complete differentiation correlates with increased insulin resistance and leads to a cavalcade of metabolic derangements. Thus by decreasing the expression of C/EBP a positive effect on insulin resistance may be achieved. An assay for the expression of C/EBPα may accordingly further substantiate an effect on insulin resistance.

By performing the above-mentioned *in vivo* studies together with the subsequent investigation of effect on gene expression it can be assayed weather a substance for example prevents and/or reduces insulin resistance or affects diseases associated with insulin resistance.

By affecting a large subset of key factors of for example the insulin resistance syndrome, i.e. improving blood glucose and insulin sensitivity, reducing triglyceride concentration, and reducing body weight, the insulin resistance syndrome, i.e. the metabolic syndrome, may be treated. Accordingly, the substances of the present invention, e.g., isosteviol and steviol, are therefore useful for the preparation of a medicament for the treatment of insulin resistance or diseases associated with insulin resistance, preferably in a human subject.

### Clinical Trials

The ongoing clinical trial is divided in two projects; project 1, wherein the dose-response relationship is determined, and project 2, wherein the long-term effect is determined. Both projects includes type-2 diabetic patients.

Project 1 is an acute, controlled, double blind, randomised study to determine the daily dosage to be used in the long-term study. The aim of project 2 is to determine the long-term effects of isosteviol on the glycemic control, blood pressure, lipid profile and insulin sensitivity in type-2 diabetic patients. See example 6.

### Formulations

For use in the present invention the substances may be administered alone, but will generally be administered in admixture with suitable pharmaceutical excipients, diluents or carriers selected with regard to the intended route of administration and standard pharmaceutical practice.

For example, the substances to be used in accordance with the invention can be administered orally, buccally or sublingually in the form of tablets, capsules (including soft gel capsules), ovules, elixirs, solutions or suspensions, which may contain flavouring or colouring agents, for immediate-, delayed-, modified-, sustained-, dual-, controlled-release or pulsatile delivery applications. The compounds of the invention may also be administered via fast dispersing or fast dissolving dosage forms.

Tablets may contain excipients such as microcrystalline cellulose, lactose, sodium citrate, calcium carbonate, dibasic calcium phosphate, glycine, and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatine and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols.

Modified release and pulsatile release dosage forms may contain excipients such as those detailed for immediate release dosage forms together with additional excipients that act as release rate modifiers, these being coated on and/or included in the body of the device. Release rate modifiers include, but are not exclusively limited to, hydroxypropylmethyl cellulose, methyl cellulose, sodium carboxymethylcellulose, ethyl cellulose, cellulose acetate, polyethylene oxide, Xanthan gum, Carbomer, ammonio methacrylate copolymer, hydrogenated castor oil, carnauba wax, paraffin wax, cellulose acetate phthalate, hydroxypropylmethyl cellulose phthalate, methacrylic acid copolymer and mixtures thereof. Modified release and pulsatile release dosage forms may contain one or a combination of release rate modifying excipients. Release rate modifying excipients may be present both within the dosage form i.e. within the matrix, and/or on the dosage form, i.e. upon the surface or coating. Fast dispersing or dissolving dosage formulations (FDDFs) may contain the following ingredients: aspartame, acesulfame potassium, citric acid, croscarmellose sodium, crospovidone, diascorbic acid, ethyl acrylate, ethyl cellulose, gelatin, hydroxypropylmethyl cellulose, magnesium stearate, mannitol, methyl methacrylate, mint flavouring, polyethylene glycol, fumed silica, silicon dioxide, sodium starch glycolate, sodium stearyl fumarate, sorbitol, xylitol. The terms dispersing or dissolving as used herein to describe FDDFs are dependent upon the solubility of the drug substance used i.e. where the drug substance is insoluble a fast dispersing dosage form can be prepared and where the drug substance is soluble a fast dissolving dosage form can be prepared.

In general a tablet formulation could typically contain between about 5 mg to about 1500 mg of a substance for use in accordance with the present invention (or a salt, solvate or prodrug thereof) whilst tablet fill weights may for example range from 50mg to 3000mg. An example formulation for a tablet is illustrated here:

| Ingredient | %w/w |
|---|---|
| Steviol, Isosteviol, or salts, solvates or prodrugs thereof | 10.000* |
| Lactose | 64.125 |
| Starch | 21.375 |
| Croscarmellose Sodium | 3.000 |
| Magnesium Stearate | 1.500 |

| | |
|---|---|
| * This quantity is typically adjusted in accordance with the desired dosage. | |

Another example formulation is illustrated here:

| Ingredient | Amount, mg |
|---|---|
| Isosteviol | 100* |
| Starch | 259 |
| Lactose | 259 |
| Magnesium stearate | 3.3 |
| Talc | 29.7 |

| | |
|---|---|
| *This quantity is typically adjusted in accordance with the desired dosage | |

The above example formulations may further contain e.g. colour, flavour or a coating in order to disguise an unpleasant taste.

As mentioned above, the daily dosage of the substances selected from the group consisting of steviol and isosteviol, or pharmaceutically acceptable salts, solvates or prodrugs thereof will be from about 0.06 to about 20 mg/kg (in single or divided doses), preferably in a range from about 1,5 to about 14 mg/kg, and more preferably from about 7 to about 14 mg/kg. Thus, tablets or capsules will for example contain 5 mg to 1,5 g of substance for administration singly or two or more at a time, as appropriate.

For aqueous suspensions and/or elixirs, the substances of the invention, or the pharmaceutically acceptable salts, solvates or prodrugs thereof, may be combined with various sweetening or flavouring agents, colouring matter or dyes, with emulsifying and/or suspending agents and with diluents such as water, ethanol, propylene glycol and glycerin, and combinations thereof.

The substances for use in accordance with the invention can also be administered parenterally, for example, intravenously, intra-arterially, intraperitoneally, intrathecally, intraventricularly, intraurethrally, intramuscularly or subcutaneously, or they may be administered by infusion techniques. For such parenteral administration medicaments are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

The substances for use in accordance with the invention can also be administered intranasally or by inhalation and are conveniently delivered in the form of a dry powder inhaler or an aerosol spray presentation from a pressurised container, pump, spray or nebulizer with the use of a suitable propellant, e.g. dichlorodifluoromethane, trichlorofluoromethane, dichlorotetra- fluoro-ethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray or nebulizer may contain a solution or suspension of the active substance, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of substance for use in accordance with the invention and a suitable powder base such as lactose or starch. The substances for use in accordance with the invention may also be formulated for delivery via an atomiser. Formulations for atomiser devices may contain the following ingredients as solubilisers, emulsifiers or suspending agents: water, ethanol, glycerol, propylene glycol, low molecular weight polyethylene glycols, sodium chloride, fluorocarbons, polyethylene glycol ethers, sorbitan trioleate, oleic acid.

Alternatively, the substances for use in accordance with the invention can be administered by the rectal or topical route. This may be in the form of a suppository, or by topical application in the form of a gel, hydrogel, lotion, solution, cream, ointment, dusting powder or skin patch. For application topically to the skin, the substances can be formulated as a suitable ointment containing the active compound suspended or dissolved in, for example, a mixture with one or more of the following: mineral oil, liquid petrolatum, white petrolatum, propylene glycol, polyoxyethylene, polyoxypropylene compound, emulsifying wax and water. Alternatively, the substances can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters, wax, cetearyl alcohol, 2-octyldodecanol, benzyl alcohol and water.

The substances for use in accordance with of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

In addition to the above described formulations, medicaments containing a substance for use in accordance with the present invention may furthermore be prepared by conventional techniques, e.g. as described in Remington: The Science and Practice of Pharmacy 1995, edited by E. W. Martin, Mack Publishing Company, 19th edition, Easton, Pa.

### Further aspects of the invention

A further aspect of the present invention relates to the use of isosteviol and/or steviol and/or their pharmaceutically acceptable analogues and/or their pharmaceutically acceptable derivates, in the manufacture of a composition for the treatment of diseases associated with insulin resistance. In accordance with the present invention, diseases associated with insulin resistance may for example be selected from the group comprising diabetes mellitus (type 2), hypertension, combined hyperlipidemia and central obesity, hyperglycemia, hyperinsulinemia and polycystic ovarian syndrome.

A further aspect of the present invention relates to the above-mentioned use of steviol and/or isosteviol for the treatment of diseases associated with insulin resistance, wherein the composition further comprises at least one soy protein source. The inventors have shown that intake of soy protein have a beneficial effect on insulin resistance and insulin resistance related disorders, such as for example body weight, LDL cholesterol, cardiovascular risk markers in general, such as e.g., arterial fatty streaks, blood lipid levels, hypercoagulability, obesity, endothelial dysfunction, microalbuminuria, hypertension etc.

Recently, the inventors of the present invention have demonstrated that soy also has a beneficial effect on LDL cholesterol and other cardiovascular risk markers in type II diabetes even in individuals with near normal lipid values [Hermansen et al., 2001]. Intake of soy has previously been linked to improved blood lipids levels and decreased arterial fatty streaks, thereby reducing the risk of developing atherosclerosis [Adams et al., 2005; Blair et al., 2002]. However, the physiological mechanism by which soy may improve blood lipid profiles has been the subject of speculations. It is unclear which soy components may contribute to the lipid-lowering property, and numerous studies have been conducted to determine which compounds of soy exert bioactive effects [Blair et al., 2002; Marc, 2005].

Soy components include protein, lipids, fibre and phytochemicals, including isoflavones. The soy protein to be used in accordance with the present invention is preferably isolated soy protein in an amount of at least 50 weight percent of the total protein content in the composition. Methods are available today, which provide soy protein products with high, fixed levels of naturally occurring isoflavones. The isoflavones for use in the present invention can be used in the glucoside and/or aglycone forms and can be included in a medicament in accordance with the present invention as part of the soy protein protein and/or by themselves and/or as part of any other medicament comprising isoflavones.

In another embodiment steviol and/or isosteviol is further combined with other components capable of reducing insulin resistance, including, but not limited to isoflavones, see e.g. Japanese patent application No. JP 2003-286166. In one embodiment a medicament to be used in accordance with the present invention, further comprises at least one isoflavone. In a preferred embodiment the at least one isoflavone is selected from the group consisting of genistein, daidzein, glycitein and equol.

The use in accordance with the present invention may further be combined with dietary fibres. This could e.g. be as a mixture of insoluble fibres and water-soluble fibres, as such soluble fibres have a lowering effect on blood cholesterol levels. The dietary fibres used in the present invention are preferably soybean fibres, more preferably soy cotyledon fibres. Such fibres are derived from dehulled and defatted soybean cotyledon and are comprised of a mixture of soluble and insoluble fibres. Soy cotyledon fibres are distinctly different from soybean fibres derived from soy hulls as well as other fibre sources. Soy cotyledon fibres are bland tasting, contain no cholesterol, are low in fat and sodium, and they have good water-binding properties and low caloric content.

A composition comprising steviol and/or isosteviol, or pharmaceutically acceptable salts, solvates or prodrugs thereof, and optionally in addition one or more of for example soy proteins, isoflavones and dietary fibres, may be given as a dietary supplementation, e.g. on a daily basis. For easy and safe administration an oral dosage form is preferred, such as e.g., tablet, capsule (including soft capsule and microcapsule), granule, powder, syrup, emulsion, suspension, sustained-release preparation and the like.

The following examples are meant to illustrate the invention further, but are in no way intended to be a limitation of the scope of the invention.

### Examples

### Abbreviations:

- GSIS: Glucose stimulated insulin secretion
- T2DM: Type 2 diabetes mellitus
- C/EBPalpha: CCAAT/enhancer binding protein (C/EBP), alpha
- ISV: Isosteviol
- Neurod1/Beta2: neurogenic differentiation 1
- IR: insulin receptor
- Ipf1/Pdx1: insulin promoter factor 1, homeodomain transcription factor
- Pbef1/visfatin: pre-B-cell colony-enhancing factor 1
- 11-beta-HSD1: 11-beta-hydroxysteroid dehydrogenase 1
- Insl: insulin I
- Ins2: insulin 2
- Akt1: thymoma viral proto-oncogene 1
- Foxa2: forkhead box A2
- Nkx2-2: NK2 transcription factor related, locus 2
- Nkx6-1: NK6 transcription factor related, locus 1
- Pax6: paired boxgene 6
- GK: Goto-Kakizaki

### Example 1:

### In vivo study of plasma concentrations of insulin, glucose and triglycerides in mice after administration of isosteviol or soybean protein

The following experiment was performed to investigate the effect of isosteviol (ISV) and soybean protein on plasma concentrations of insulin, glucose and triglycerides. Four experimental groups, each consisting of 10 KKAy-mice (obtained from Clea Japan, Tokyo, Japan) and one control group of 20 C57 mice (obtained from Taconic, Ry, Denmark), all 5 weeks old, were feed the following three test diets in a period of nine weeks.
A: standard chow diet (SCD)(ALTROMIN 1320, Brogaarden, Hørsholm). Diet A was fed to one experimental group and to the control group.
B: standard chow diet (SCD) (ALTROMIN 1320, Brogaarden, Hørsholm) + 0.02 g/kg BW of ISV (Waco Chemicals, Osaka, Japan).
C: diet containing 50 weight-% standard chow + 50 weight-% Soybean Protein (SBP) (NutriPharma, Oslo)

Hormones and lipids were measured from blood sample at start and end of the treatment period. Fasting blood glucose as well as body weight were measured once a week. The body weight between the diabetic groups did not differ. The blood was taken from the tail of the animals.

Isosteviol (ISV) and Soybean Protein (SBP) were given orally. The SBP were incorporated in the food pellet and the ISV was dissolved in absolute ethanol, the solution was inoculated on the pellets and the ethanol was allowed to evaporate, leaving the ISV on the food pellet. All diets were adjusted for vitamins and minerals, so that all diets content the same amount. Beside that, water was given ad libitum and a light cycle of 12 hours/12 hours was used. The experiment was performed in accordance with the guidelines of the Danish Council on Animal Care.

### Results:

### Plasma insulin concentration

As is evident from figure 2, no difference in the plasma insulin concentration between the different groups was seen at the onset of the study (column 1, 3, 5 and 7). After 9 weeks of treatment the KKAy-mice treated with ISV (column 6) and KKAy-mice treated with SBP (column 8) showed decreased plasma insulin concentration compared to KKAy-mice that received the standard chow diet (column 4). SCD vs. ISV showed a 192 % reduction (p<0.05) and SCD vs. SBP showed 420 % reduction (p<0.01)), respectively.

These results indicate that treatment with either ISV or SBP increases insulin sensitivity. Isosteviol and/or SBP is therefore useful in order to prevent and/or reduce insulin resistance and treat diseases associated with insulin resistance, such as e.g., diabetes mellitus (type 2), hypertension, hyperlipidemia and obesity.

### Plasma glucose concentration

Just as for the plasma insulin concentration, no difference in the plasma glucose concentration was seen at the onset of the study, see figure 3; column 1, 3, 5 and 7. After 9 weeks of treatment the KKAy-mice treated with ISV (column 6) and KKAy-mice treated with SBP (column 8) showed a decreased plasma glucose concentration compared to the KKAy-mice that received the standard chow diet (SCD)(column 4). SCD vs. ISV exhibited a 73 % reduction (p<0.01) and SCD vs. SBP and 188 % reduction (p<0.01), indicating that the increased insulin sensitivity triggered the normal metabolic activities, effectively reducing the glucose concentration.

For KKAy-mice receiving treatment with SBP, i.e. mice receiving diet C, the plasma glucose concentration at the end of the treatments period corresponded to plasma glucose concentrations in healthy C-57-mice.

### Plasma triglyceride concentration

Plasma triglyceride (TG) concentration was significantly lowered for the groups treated with ISV by 101 % (p<0.01) and SBP by 61 % (p<0.05), see figure 4.

Earlier studies have shown, that insulin resistance in rodents and humans is associated with increased triglyceride deposition within skeletal muscle cells (Storlien *et al*., 1991) thus the lowering of the Plasma triglyceride concentration indicates a positive effect on the insulin resistance.

### Example 2:

### Gene expression in islets from diabetic KKAy-mice

As it is thought that the insulin pathway can have relevance in the development of insulin resistance, the expression pattern of a number of gene involved in the insulin pathway or otherwise related to insulin resistance have been examined in islets from diabetic KKAy-mice, after stimulation with isosteviol (ISV) and soybean protein (SBP). Such genes are furthermore candidate genes for identifying individuals at risk for the development of insulin resistance or to develop new pharmacological agents. At present there are few reliable methods for presymptomatic diagnosis of a genetic predisposition for e.g. type II diabetes. Therefore, the present invention also enables the design of genetic based tests for predicting and detecting the onset of insulin resistance.

### Protocol

A the end of the nine week period of the *in vivo study* in example 1 RNA was purified from Islets, muscle, liver and fat from the three experimental groups, using standard techniques as described in Kiergen, RNA easy.

Gene expression of PDX-1, IRS1, Beta2, 11beta-HSD-1, Akt1, C/EBP-alpha, CPT1, GLUT2, IGF1, IR and Ins1 was measured with Real time RT-PCR using Taq Man Assays normalized to 18S rRNA, using the standard protocol (ABI, USA). All reagents were obtained from (ABI, USA). All probes are standard probes obtainable from ABI, USA, under the specific probe code number, given in respect of each gene.

### PDX 1

### Probe used: Mm00435565_m1

PDX-1 is a homeodomain transcription factor essential for pancreatic development and mature beta cell function and plays a key role in normal insulin secretion by islets. (Edlund, 1998). The PDX-1 gene is initially expressed in exocrine and endocrine pancreatic precursors but later becomes restricted mainly to β cells. Loss of PDX-1 expression leads to pancreatic agenesis and haploinsufficiency of the PDX-1 gene results in defects in glucose-stimulated insulin secretion in humans and mice (Ahlgren et al., 1998).

As shown in figure 5, islets from KKAy-mice treated with ISV showed an increased expression of PDX-1 compared to KKAy mice treated with the standard diet (96%, p<0,05).

As previously discussed herein, some recent studies indicate that mutations in PDX-1 may predispose individuals to late onset type 2 diabetes due to insulin resistance; (Hansen et al., 2000). These studies indicates, that lowering the PDX expression may contribute to the development of type II diabetes by causing impaired expression of GLUT-2 and insulin. Thus an increase in expression of PDX-1 after treatment with isosteviol indicates that isosteviol is capable of preventing or reducing insulin resistance by stimulating PDX-1 expression.

### GLUT-2

### Probe used: Mm00437443_m1

GLUT 2 is a transmembrane protein which is involved in passive transport of glucose over cellular membranes e.g. the liver and pancreatic β-cells. The receptor is insulin independent.Glucose-stimulated insulin secretion by the β-cells is a highly regulated process in which GLUT-2 and glucokinase (GK) have been proposed to play important roles.

As is evident from figure 6, showed islets from KKAy-mice treated with either ISV or SBP a significant increased expression of GLUT-2 compared to KKAy mice treated with the standard diet (60%, p<0,05), (229 %, p < 0,001), respectively.

As GLUT 2 is involved in the passive transport of glucose over cellular membranes e.g. the liver and pancreatic β-cells, the significant increased expression of GLUT-2 after stimulation with either isosteviol or soybean protein, indicates that isosteviol or soybean protein improves glucose sensitivity, i.e. a reduces the insulin resistance.

### BETA2

### Probe used: Mm01280117_m1

Beta2 is an important regulator of insulin gene expression and is expressed in pancreatic endocrine cells, the intestine, and the brain. Resent studies indicate, that impairment of the Beta2 expression relates to insulin resistance.

Figure 7, shows islets from KKAy-mice treated with either ISV or SBP a significant increased expression of beta2 compared to KKAy mice treated with the standard diet; (60%, p<0,05) and (78%, p<0,05), respectively.

As previously discussed herein, a significant increase in the overall expression of beta2 after treatment with isosteviol and soybean protein respectively, suggests that isosteviol and soybean protein are capable of reducing insulin resistance.

### IGF-1

*Probe: Mm00439561_m1*

Insulin-like growth factor 1 (IGF-1) is a polypeptide protein hormone similar in molecular structure to insulin. It plays an important role in childhood growth and continues to have anabolic effects in adults. Usala et al, 1992, found that diabetic patients with extreme insulin resistance have substantial improvement in metabolic control during administration of IGF-1.

As shown in figure 8, islets from KKAy-mice treated with ISV and SBP showed a decreased expression of IGF1 compared to KKAy mice treated with the standard diet (46%, p<0,05) and (61%, p<0,05), respectively.

These results indicate a potential positive effect on the risk of developing angiogenesis and microvascular complications e.g. reduced risk of diabetic retinopathy.

### 11beta-HSD1

*Probe: Mm00476182_m1*

11-Beta Hydroxysteroid Dehydrogenase (11 beta-HSD1) is the name of a family of enzymes that catalyzes the conversion of inert 11 keto-products (cortisone) to active cortisol, or vice versa, thus regulating the access of glucocorticoids to the steroid receptors. 11beta-HSD1 is widely expressed, particularly in the liver.Glucocorticoids play a major role in glucose homeostasis by influencing hepatic gluconeogenesis and glycogen degradation. Genetic deletion of 11beta-HSD1 lowers plasma glucose levels in mice on high-fat diets and attenuates the activation of enzymes involved in hepatic gluconeogenesis suggesting that inhibitors of this enzyme may be of use in various metabolic disorders. Many glucocortioids effects directly oppose the effects of insulin, thereby inducing insulin resistances. For examble, glucocorticoids impair insulin dependent glucose intake in the peripheral tissue, enhanceing glucose production in the liver, inhibiting insulin secretion from pancreatic beta-cells.

As shown in figure 9, islets from KKAy-mice treated with ISV and SBP showed a significant decreased expression of 11beta-HSD1 compared to KKAy mice treated with the standard diet (61%, p<0,001) and (61%, p<0,05), respectively.

The 61% down regulation of 11beta-HSD-1 by ISV is potentially very interesting since 11beta-HSD-1 expression and activity are increased in islets of diabetic ZDF rats and Troglitazone concomitantly prevents both the increase in 11beta-HSD-1 and diabetes development. Furthermore, as the activator of the glucocortioids is downregulatated by administration of isosteviol, this will reduce the glucocorticoids detrimental effect on insulin resistance, thereby increasing insulin sensitivity.

### INS-1

*Probe: Mm01259683_g1*

Whereas humans have one insulin gene, the mouse has two insulin genes Ins1 and Ins2.

As shown in figure 10, islets from KKAy-mice treated with ISV and SBP showed a significant increased expression of INS-1 compared to KKAy mice treated with the standard diet (132%, p<0,5) for both ISV and (135%, p<0,5) for SBP, indicating that isosteviol and a soybean protein based diet has a positive effect on the insulin secretion.

### C/EBP-alpha

### Probe: Mm00514283_s1

The CCAAT/enhancer-binding protein (C/EBP) family of transcriptional regulators is critically important for the activation of adipogenic genes during differentiation. Glucose and insulin in excess are capable of down-regulating CCAAT enhancer binding protein α (C/EBPα), a transcription factor essential for maintenance of a fully differentiated adipocyte phenotype (2-4). (Reusch and Klemm; 1999)This is an important observation because loss of complete differentiation correlates with increased insulin resistance and leads to a cavalcade of metabolic derangements.

As shown in figure 11, islets from KKAy-mice treated with ISV and SBP showed a significant decreased expression of C/EBPαcompared to KKAy mice treated with the standard diet (49%, p<0,01) for ISV and (56%, p<0,5) for SBP, indicating that isosteviol and a soybean protein is capable of decreasing insulin resistance.

Taken together, the above experiments demonstrated that oral administration of isosteviol and soybean protein prevents and/or reduces insulin resistance. Isosteviol and soybean protein would therefore be suitable as a component in a dietary supplement for patients having - or are at a risk of developing - diseases associated with insulin resistance, such as diabetes mellitus (type 2), hypertension, hyperlipidemia and central obesity, as isosteviol and soybean protein might slow the development of said complications.

### Example 3

### In vivo study of plasma concentrations of insulin, glucose and triglycerides, and effects on gene expression, after administration of isosteviol to mice

The aim of the following experiment was to investigate the beneficial effects of isosteviol on the metabolism by investigating the effect of isosteviol (ISV) on the plasma concentration of insulin, glucose and triglycerides, and on body weight. The experiment furthermore investigated the long-term effect of isosteviol on the gene expression profile of key insulin regulatory genes in islets, i.e. the long-term effect of isosteviol on insulin resistance.

### Materials and Methods

### Animals:

Twenty six male KKAy-mice (Clea Japan, Tokyo, Japan), all 5 week-old, weighing 22 - 25 g were randomized to 2 groups and treated for 9 weeks with;
A: standard chow diet (control);
B: standard chow diet + 20 mg/kg BW of Isosteviol (hereinafter ISV).

The composition of the standard chow diet dry matter was: Protein 24%, carbohydrate 71%, and Lipids 5%. ISV (ent-16-ketobeyeran-19-oic acid; Wako Pure Chemical Industries, Osaka, Japan) was incorporated in the mice food pellet and administered orally.

As a non-diabetic control group (C), twenty normal C57/BL-mice (Taconic, Ry, Denmark) were fed with standard chow diet (control to A).

For *in vitro* studies with steviol and ISV, we included adult female NMRI mice (Taconic, Ry Denmark), all weighing 22 - 25 g. We used 12 hours light/dark cycle. The Danish Council for Animal experiments has approved the study.

### Plasma analysis:

Hormones and lipids were measured from blood sample at start and end of the treatment period. Blood sample was taken from the tail of the animals on chilled tubes containing heparin/aprotinin and centrifuged (4000 g, 60 seconds, 48C), and plasma was frozen for subsequent analysis of insulin, glucose and triglycerides. Fasting blood glucose, as well as BW, was measured before and after intervention.

### Assays:

Plasma blood glucose was determined using the glucose oxidase method (GOD-PAP, Boehringer Mannheim, GmbH Germany). Insulin was determined by radioimmunoassay with a guinea pig antiporcine insulin antibody (PNILGP4, Novo Nordisk, Bagsværd, Denmark), and mono- [1251]-(Tyr A14) labelled human insulin (Novo Nordisk) as tracer and rat insulin (Novo Nordisk) as standard. We separated free and bound radioactivity using ethanol (Heding, 1972). Interassay and intra-assay variation was below 10%. ISV did not interfere with the insulin assay at the concentrations studied. Triglycerides was determined using colorimetric kits (Roche Diagnostics, Boehringer Mannheim, GmbH Germany). The fasting glucose-insulin index was calculated as the product of plasma insulin and plasma glucose ([plasma insulin ng/ml]x[plasma glucose mmol/l]/1000) and compared between groups as relative units (Chang et al., 2005).

### Insulin content:

The Insulin content of isolated islet from the KKAy mice was measured using RIA as described for the Insulin assay. Total islet protein was purified from the organic phase during from the RNA Trizol preparation according to the manufactures instruction (Gibco BRL, Life Technologies, Roskilde, DK).

### Islet isolation:

Islets were isolated by the collagenase digestion technique. In brief, the animals were anesthetized with pentobarbital (50 mg/kg intraperitoneally) and midline laparotomy was performed. The pancreas was retrogradely filled with 3 mL ice-cold Hanks balanced salt solution (([HBSS] Sigma Chemical, St Louis, MO) supplemented with 0.3 mg/ml collagenase Boehringer Mannheim GmbH,Germany). The pancreas was subsequently removed and incubated for 19 minutes at 37 °C. After rinsing in HBSS, the islets were handpicked under a stereomicroscope and immediately transferred to a tubes containing 1 ml Trizol (Gibco/Invitrogene, Calsbad, CA, USA). The islets isolated from the NMRI mice were incubated overnight at 37°C and 95% normal atmosphere/5% CO₂ in 10 ml RPMI 1640 containing 11.1 mmol/L glucose supplemented with penicillin G and 100 µg/mL streptomycin (all Gibco/Invitrogene, Calsbad, CA, USA).

### Isolation of RNA:

For each group, islets from 3-4 mice (180-200 islets) were pooled in 1 ml Trizol reagent (Gibco/Invitrogene, Calsbad, CA, USA) before RNA purification. Total RNA was extracted according to the manufactures' instructions. RNA was quantified by measuring absorbance at 260 and 280 nm. The quality of the RNA was checked by the Agilent 2100 bioanalyzer (Agilent, Santa Clara, USA).

### Real-time RT-PCR:

The expression of Pdx-1, Akt1, GLUT2, C/EBPalpha, CPT1, 11-Beta-HDS1, IR, Visfatin, Beta2, IRS1, Ins1 and Ins2 were investigated by real-time RT-PCR. cDNA was synthesized using IScript (BioRad, Hercules, CA, USA) according to the manufacturer's instructions. Total RNA at 50 ng per 10 uL of reaction mixture was used for measurement of the target mRNA. The real-time RT-PCR assay was performed using the ABI 7500 FAST machine (ABI; Foster City, CA). 10 ul real-time RT-PCR reactions consists of 5 ul 2x TaqMan® FAST Universal Master Mix (P/N 43660783, ABI; Foster City, CA), 0.5 ul 20xTaqMan® Assay/probe (ABI; Foster City, CA)) and cDNA equivalent to 50 ng of total RNA in 4.5 ul H₂O. Thermal FAST cycle program was: 20 s at 95 °C followed by 40 cycles of 3 s at 95 °C and 30 s at 60 °C. Reactions were set up in triplicate for each sample, and gene expressions were normalized to eukaryotic 18S rRNA expression (assay Hs99999901_s1; ABI; Foster City, CA). All assays were carried out in 96-well format plates covered with optical adhesive cover (P/N 4346906 and P/N 4311971 ABI; Foster City, CA). We used the 2-ΔΔCT method to calculate the relative gene expression (as described in User Bulletin 2, 1997, from Perkin-Elmer Corp. detailing the aspect of relative quantization of gene expression). TaqMan® Assays used were: Pdx-1/Ipf1 (assay Mm00435565_m1), Akt1 (assay Mm00437443_m1), GLUT2 (assay Mm00446224_m1), C/EBPalpha (assay Mm00514283_s1), Cpt1 (assay Mm00550438_m1), 11-Beta-HDS1 (assay Mm00476182_m1), IR (assay Mm00439693_m1), Visfatin/pbef1 (assay Mm00451938_m1), Beta2/NeuroD (assay Mm01280117_m1), IRS1 (assay Mm00439720_s1), Ins2 (assay Mm00731595_gh) and Ins1 (assay Mm01259683_g1).

### Affymetrix microarray analysis:

cRNA preparation and in vitro transcription: In total 6 Affymetrix arrays MOE430 2.0 probe array cartridge were used i.e. for analysis of 3 replicas for the KKAy control group and the ISV group, respectively. Each of the 6 samples consisted of pooled RNA from 3-4 KKAy mice. Preparation of target for hybridization was prepared from 340ng of total RNA using MessageAmp™ II aRNA Amplification Kits (Ambion), according to the manufacturers instructions. Arrays were hybridised and scanned as described elsewhere (Kruhoffer et al., 2005).

### Incubation of islets:

The NMRI female mice islets were rinsed twice with a modified Krebs-Ringer buffer (KRB) supplemented with 3.3 mmol/L glucose and 0.1% human serum albumin (Sigma). The KRB contained 125 mmol/L NaCl, 5.9 mmol/L KCI, 1.2 MgCl, 1.28 CaCl₂ and 25 mmol/L HEPES (Ph 7.4: all Sigma). Single islets were incubated in 100 µL KRB containing glucose (3.3 and 16.7 mmol/L) and steviol/ISV according to the protocols (Jeppesen et al., 2000). After incubation (60 min), 50 µL of the medium was frozen for RIA analysis of insulin.

### Statistical analysis:

Data are expressed as means ± standard error of the mean (SEM). Statistical significance between two groups was evaluated using a two-tailed t test. A p value of less than 0.05 was considered statistically significant. For gene expression analysis one-way ANOVA was applied.

### Results

### Metabolic effects of ISV.

At the start of the intervention study, no significant difference was found in blood glucose or insulin levels between groups (Fig. 12A, 12B). Importantly, there was no difference between the non diabetic C57/BL-mice control group and the diabetic KKAy control group at age 5 weeks, ensuring that the KKAy mice have not developed insulin resistance before intervention was started.

After the 9-week treatment period, the KKAy control group developed a significant increase in plasma glucose of 181% (9.4 vs. 26.3 mmol/l, p<0.001), whereas the non-diabetic C57/BL- control group only experienced an increase of 10% (8.6 vs. 9.5 mmol/l, p=0.013). The plasma insulin increased correspondingly 266 fold (0.9 vs. 234.1 ng/ml p<0.001) and 4.5 fold (0.4 vs. 1.8 ng/ml p<0.001) for KKAy and C57/BL, respectively. The plasma glucose and insulin in the KKAy group were 2.8 fold (9.5 vs. 26.3 mmol/l, p<0.001) and 130 fold (1.8 vs. 234.1 ng/ml, p<0.001) higher, respectively, compared to the C57/BL control group (Fig. 12A, 12B). While plasma glucose for the KKAy control group was increased 2.8 fold at the end of the study, the ISV treated KKAy mice experienced only a 1.8 fold increase (8.9 vs. 16.2 mmol/l, p=0.003) (Fig. 12A). Plasma insulin for the KKAy group was increased 266 fold during the study period, whereas the ISV treated KKAy mice only increased 81 fold (1.0 vs. 80.9 ng/ml p=0.001) (Fig. 12B). Using KKAy at 5 weeks as baseline, treatment with ISV resulted in a 59% (p<0.01) reduction in plasma glucose compared to the increase for the KKAy. Compared to the KKAy control, ISV treatment reduced plasma insulin increase by 62% (p<0.05).

The glucose-insulin index (as a measure for the insulin resistance) was, as expected, higher for the KKAy control and ISV group compared to the non diabetic C57/BL group (3.4 vs. 8.3 unit, p<0,05 and 3.4 vs. 8.6, p<0.05 respectively) and there was no difference between the KKAy control and ISV group (8.3 vs. 8.6, p=0.88)(Fig. 12C). At the end of the study there was a marked decrease in the glucose-insulin index for the ISV group compared to the KKAy control (6932 vs. 1596 unit, p<0.01), illustrating that ISV has decreased the insulin resistance (fig. 3C).

### Changes in lipid levels.

At age 14 weeks the TG level was increased by 192% for the KKAy control group compared to age 5 week (1.34 vs. 3.92 mmol/l, p<0.001), while the ISV group had increased by 62% (1.25 vs. 2.02 mmol/l, p=0.003)(Fig. 13) i.e. ISV reduced the plasma TG increase in the KKAy by 74 % (p<0.01).

### Body weight

At the start of the intervention study, no significant difference was observed in body weight between the KKAy control and the ISV groups (Fig. 14). ISV caused a weight reduction both after 5 weeks (KKAy control 38.6 g vs. ISV 29.9 g, respectively) and after 9 weeks treatment (KKAy control 41.6 g vs. ISV 36.3 g, respectively). The reduction in weight achieved was 13% (p<0.001) for the ISV group at the end of the 9 week treatment period (Fig. 14).

### Gene expression analysis in islets

### Relative Real Time RT-PCR.

Analysis of islets from pancreas of KKAy control and ISV treated mice: RNA was isolated and subsequently analysed for 12 genes (Fig. 15 and 16), related to insulin secretion and regulation, for changes in their transcript abundance (TA). The change in TA was calculated for the ISV group compared to the non-treated control group (Fig. 15 and 16). Islets from the ISV group vs. the control group experienced an increased expression of *Pdx1*/*Ipf1* (96%, p<0,025), *Beta2*/*neuroD* (60%, p<0,025), *GLUT2* (201%, p<0,001), *Ins1* (132%, p<0,025) and decreased expression of *C*/*EBPalpha* (49%, p<0,01) and *11-beta-HSD-1* (61%, p<0,001).

The ISV treated mice have a more than 3 fold up regulation of the *GLUT2* glucose transporter protein gene transcript suggesting that the glucose sensitivity is increased. The observed increase in Ins1 expression of more than 2 fold for the ISV group correlates well with the increase in *GLUT2.* The upregulation of the bHLH transcription factor *Beta2*/*NeuroD,* a key regulator of both insulin genes transcription in pancreatic beta-cells in which heterozygous mutations is found associated with the development of T2DM, also support the increase in Ins1 expression. For the ISV group another central pancreatic transcription factor, the pancreatic duodenal homeobox gene-1 *(Pdx1)* is up regulated nearly 2 fold. Of particular interest is the 2.5 fold down regulation of 11beta-hydroxysteroid dehydrogenase-1 (11-beta-HSD-1), encoding the enzyme that metabolizes inactive precursors to active glucocorticoid (GC) hormones. The observed increase in plasma insulin correlates well with the detected reduction in *11-beta-HSD-1* mRNA level for the ISV group. Interestingly, we detected a 2 fold reduction of the transcription factor *C*/*EBP-alpha* (known in liver to bind to the *11-beta-HSD-1* gene promoter and act as a positive regulator of *11beta-HSD1*) mRNA expression (Bruley et al., 2006).

### Affymetrix Gene chip analysis.

The important changes in islets gene expression prompted to conduct gene chip analysis on the KKAy control and ISV groups. The array analysis supported the real time RT-PCR analysis, indicating an upregulation of *GLUT2, Beta2*/*neuroD* and *Pdx1* expression, down regulation of C/EBPalpha, 11-beta-HSD-1 and no changes in *IR, visfatin, InsII and Akt1.* Surprisingly, the array analysis did not show any statistical significant increase in *InsI* expression. One explanation for this may be that the insulin mRNA is so abundant in the islets RNA preparation that the dynamic range of the array was exceeded. Furthermore, the array data demonstrated an increased expression in Foxa2 (1.9 fold), Somatostatin (2.3), Pax6 (1.9), Nkx2-2 (2.3) and Nkx6-1 (2.64). FoxA2, Pax6, Nkx2.2 and Nkx6-1 are islet enriched transcription factors required for beta cell development and somatostatin functions, both acting as insulin sensitizer and as an inhibitor of insulin secretion from the pancreatic beta cells ((Henseleit et al., 2005; Tolis et al., 2006).

### Changes in KKAy islets insulin content.

The gene expression analysis showed that a number of transcription factors, including Pdx1, Beta2, Pax6 (known to bind and positively regulate insulin transcription) were up-regulated. Furthermore, the QPCR results clearly showed an increased transcription of Ins1 for the ISV group. In order to clarify if this increased levels of Ins1 mRNA are translated, the total insulin content from the very limited protein fraction collected from the organic phase during RNA preparation from isolated islets from the KKAY and ISV group was measured using RIA (Fig. 17). The insulin content from the ISV group compared to the KKAy control was 2.4 fold (p=0.003) higher, supporting the gene expression analysis.

The advanced age of the KKAy control group resulted in relatively high fasting blood glucose levels comparable with a rather severe diabetic state. In contrast, the isosteviol treated mice decreased both plasma glucose and insulin levels, indicating an improvement in insulin sensitivity.

From the above experiments and subsequent analysis of gene expression it can be concluded that the administration of isosteviol have a long-term effect on insulin resistance. Many of the above experiments give an indication of this effect; however, especially the fact that a lower plasma insulin concentration is observed together with a lower plasma glucose concentration demonstrates the improved insulin sensitivity.

### Example 4

### In vitro dose response study with isosteviol

### Materials and methods

See example 3.

### Results

Isosteviol (ISV) (10⁻¹² mol/l - 10⁻⁸ mol/l) potentiated insulin secretion stimulated by 16.7 mmol/L glucose, with an apparent maximal effect obtained in the presence of approx. 10⁻⁸ mol/l ISV (Fig. 18). ISV did not potentiate the insulin secretion at low glucose (3.3 mmol/L), illustrating that the insulinotropic effect of ISV is glucose dependent (Fig. 18).

### Example 5

### In vitro dose response comparative study with isosteviol and steviol

It has previously been demonstrated by the present inventors that steviol, exerts a potent insulinotropic effect on isolated mouse islets (Jeppesen, P.B. et al., 2000). In order to evaluate the potency between isosteviol and steviol, the following *in vitro* study was performed. At low concentration, (10⁻¹⁰ mol/l), isosteviol was significantly more potent than steviol (p<0.05). At higher concentration (10⁻⁸ mol/l - 10⁻⁶ mol/I) there was not found any significant difference between the two groups (Fig 19).

### Example 6

### Clinical trial, use of isosteviol for the treatment of insulin resistance

### Project 1: Dose-response study (0, 25, 100, 250 and 500 mg) on the post-prandial glucose, insulin, glucagon, lipid and incretin responses.

In this study 26 subjects (males and females), aged 30-70 years will be included. The study design is acute, paired, blinded, cross-over study and isosteviol is administered at four doses (25, 100, 250 and 500 mg) and placebo at random order together with a standard breakfast meal.

The first out of the six visits is a screening visit where the subjects are considered eligible for entering the study. The subsequent 5 visits are separated by 1-2 weeks and the subjects will be fasting from 11 am the day before. The subject will not take SU medication for 7 days before the individual visits. A venflon® is placed in an antecubital vein for blood sampling. Blood samples (analysed for glucose, insulin, glucagon and incretins (GIP and GLP-1)) are withdrawn before and at time points up to 360 minutes after intake of the test meal. Total diuresis will be collected (and analysed for glucose) and blood pressure will be monitored during the meal study.

### Inclusion criteria:

1. Type 2 diabetes treated with either diet or oral antidiabetic medication.
2. Diabetes duration 6 months or higher.
3. Diabetes onset > 30 years of age.
4. Fasting plasma glucose < 10 mM.
5. HbA1c>6.1 and < 9 %

### Exclusion criteria:

1. SU and insulin treatment. However, if investigator estimates that SU or insulin can be paused for 7 days before the 5 test meals this treatment is not an exclusion criterion.
2. Late diabetic complications with the exception of simplex retinopathia and microalbuminuria.
2. Treatment with medication known to alter the glucose metabolism (glucocorticoids, sedatives and stimulants).
3. Signs of renal (se-creatinin>150 mikromol/L), liver (ALAT and/or alkaline phosphatase > 3 times upper reference limit) or cardiac disease (NYHA class 3 or 4), unstable angina pectoris or AMI within 12 months before the study.
4. Alcohol abuse.
5. Hypertension (>180/110 mmHg).
6. Body mass index <20 or > 40 kg/m².

Current treatment with statins is continued during the study period at constant dose.

### Design:

The subjects will be fasting from 11 am the evening before each meal study and abstain from smoking or take any medication except for the study medication. A venflon® is placed in an antecubital vein for blood sampling (timepoints: -30, 0, 10, 20, 30, 45, 60, 90, 120, 180, 240, 300, 330 and 360 min). At t=0 the test meal (total energy 1725 kJ; 16 E% protein, 30 E% fat, 54 E% carbohydrates) is consumed within 15 minutes. Blood samples are analysed for plasma glucose, insulin, glucagon, GIP, GLP-1, FFA and triglyceride.

### Project 2: Clinical study: Long-term controlled, randomised, double-blind, parallel design study for 10 weeks in 76 type 2 diabetic subjects.

Aim: to determine the effects of a fixed dose of isosteviol on the glycemic control, blood pressure, lipid profile and insulin sensitivity in type 2 diabetes.

In this study 76 type 2 diabetic subjects aged 30-70 years will participate. From our previous acute dose response study (project 1), we will choose the optimal dose of isosteviol to be administered. The study contains a total of 9 visits. The subjects will receive isosteviol at the same dose thrice daily (before breakfast, at noon and at dinner). Twenty four hour blood pressure measurement and a meal test will be carried out at the beginning and at the end of the study. The treatment period is 10 weeks, whereas the total study period is longer. The placebo group will receive maize starch tablets. The patients will be monitored during the study according to the protocol, regarding body weight, fasting blood glucose, blood pressure and lipids. After 10 weeks treatment the patients will be exposed to an IV glucose tolerance test.

A total of nine visits are planned. The first visit is a screening to evaluate if the subject is eligible for enrolment according to in- and exclusion criteria. Hereafter the subjects will enter a "run-in" period of one week on placebo medication. The subjects will undergo two test-meals and an intravenous glucose-tolerance test (Bergmans minimal model) to estimate the insulin sensitivity (visit 7). Every second week the subjects will be monitored with measurements of blood glucose, blood pressure, lipid profile (fasting triglycerides and cholesterol) and body weight. The subjects will (except for the blood pressure monitor visits) be fasting before each visit (from 11 a.m. the evening before) and the only medication they are allowed to take is the study medication and possibly non-blood glucose lowering medication. The diabetic subjects will be asked to continue with their usual antidiabetic (with the exception of SU and insulin, see above at exclusion criterias), antihypertensive or lipid lowering agents without changing doses during the 10 weeks period.

To study the bioavailability of isosteviol and its metabolic fate, urine, faeces and plasma samples will be collected at control visits and during the meal tests. They will be extracted and analysed for the presence of isosteviol and its metabolites e.g. steviol-16, 17-α-epoxide and 15-α-hydroxysteviol by means of an Ultra Performance Liquid Chromatography-MS system. Using samples from our previously acute studies we will be able to estimate the half-life of isosteviol, (amount of isosteviol in the body (plasma concentration) to decline to half of its value), which gives us important information to be used in the long-term study. The method will also be used to describe if there is a difference in the bioavailability between the patients, and finally also to ensure compliance.

The study will be conducted in accordance with the Declaration of Helsinki and Good Clinical Practice guidelines. All study subjects will receive oral and written information about the study and will give written, informed consent before study enrolment. The first visit will include a clinical examination to see if the patient is eligible for enrolment. The patients will be instructed to fast from midnight prior to the visits. The two clinical trials have already been approved by the Danish Medicines Agency, The Danish Ethical Committee and Ministry of Interior and Health for the GMP production of the drug.

Efficacy analyses: Total cholesterol, LDL-cholesterol, TG, Diurnal blood pressure, fasting blood glucose, HbA1c, as well as glucose, insulin, glucagon, triglyceride and FFA responses to the test meal and to the IVGTT.

Procedures: 24 h blood pressure measurement (Spacelab 90207), Meal test, IVGTT. Safety variables and analyses: Incidence of adverse events and hypoglycaemic episodes. Laboratory parameters (ALAT, Creatinine, Sodium, Potassium, Haemoglobin, Leucocytes, Platelets, weight).

### Inclusion and Exclusion criteria are similar to the dose-response study (see above):

The trial will be carried out at Aarhus University Hospital, Aarhus Sygehus THG. Analysis of the blood samples (HbA1 C, glucose, insulin, glucagons, free fatty acids (FFA), triglycerides (TG), total cholesterol, LDL-cholesterol) will be carried out at the diabetes research lab, Aarhus Sygehus THG, Aarhus University Hospital. Liver tests, creatinine, S-Na, S-K will be followed.

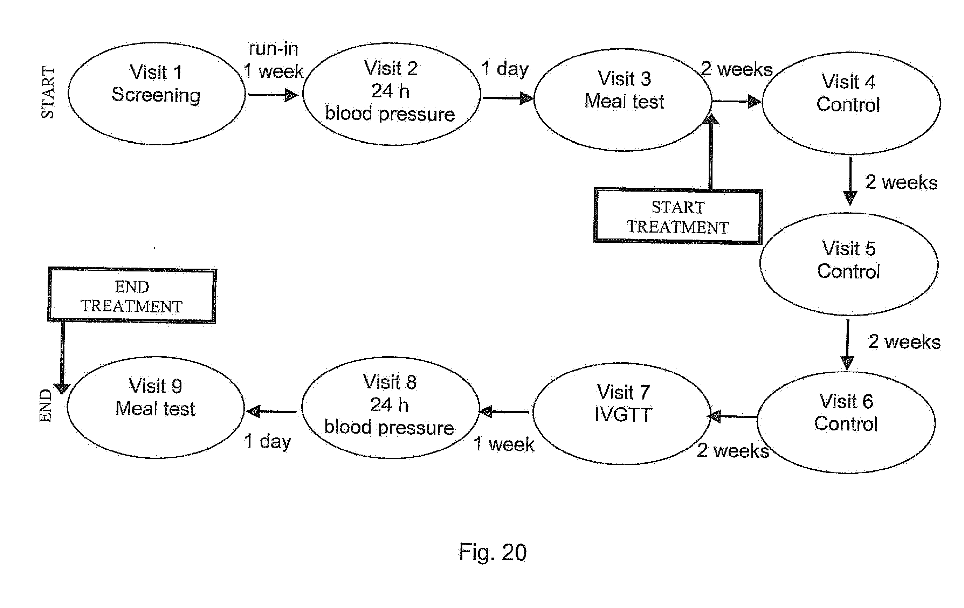

An overview of the study can be seen in figure 20.

### Meal test:

The patients will be fasting from 22.00 the night before and instructed not to smoke or take their normal medication prior to the experiment. A venflon will be placed in an antecubital vein and blood samples will be drawn at time points: -30, 0, 10, 20, 30, 45, 60, 90, 120, 180 and 240 minutes. Blood samples will be analysed for plasma: glucose, insulin, glucagon and FFA. At start and at min 240 triglycerides, FFA, total cholesterol, LDL-cholesterol will be measured. On each of the 2 experimental days approx. 80 ml of blood will be taken. The total energy content of the test meal will be 1725 KJ (protein 16 E%, fat 30 E%, carbohydrate 54 E %). Lunch will be served after finishing the test meal.

### IV glucose tolerance test:

Bergman's minimal model will be used (Bergman et al: 1986). On the experimental day a venflon will be placed in antecubital veins bilaterally, where blood samples can be drawn and glucose infused. At the beginning of the study a tablet will be taken by the patient containing either steviol or placebo. Blood samples will be taken at time points: (-15, -5, 0, 2, 4, 6, 8, 10, 12, 15, 18, 20, 25, 30, 35, 40, 60, 70, 100, 120, 140, 160, 180 minutes), 2 ½ ml will be taken for each time point, which will be analysed for glucose, insulin and glucagon. At time point 30 min, insulin will be infused (0.05 U/kg BW). During the IV glucose tolerance test approx. 70 ml of blood, will be taken at each experiment. Lunch will be served to the patients after finishing the experiment.

### Blood pressure measurement:

At all visits the blood pressure will be measured. At visit 2 and 8, a 24 hour ambulatory blood pressure measurement will be carried out (the day before the meal test).

### References

Adams MR, Golden DL, Williams JK, Franke AA, Register TC, Kaplan JR.; Soy protein containing isoflavones reduces the size of atherosclerotic plaques without affecting coronary artery reactivity in adult male monkeys. J Nutr.; (2005) Dec;135(12):2852-6.
Ahlgren U, Jonsson J, Jonsson L, Simu K, Edlund H.; beta-cell-specific inactivation of the mouse Ipf1/Pdx1 gene results in loss of the beta-cell phenotype and maturity onset diabetes. Genes Dev.; (1998) Jun 15;12(12):1763-8)
Bergman et al.; Message in Diabetes research; New York: Wiley and sons (1986) 15-34.
Blair RM, Appt SE, Bennetau-Pelissero C, Clarkson TB, Anthony MS, Lamothe V, Potter SM.; Dietary soy and soy isoflavones have gender-specific effects on plasma lipids and isoflavones in golden Syrian f(1)b hybrid hamsters. J Nutr.; (2002) Dec;132(12): 2585-91
Bruley C, Lyons V, Worsley A G, et al.; A novel promoter for the 11 beta-hydroxysteroid dehydrogenase type 1 gene is active in lung and is C/EBPalpha independent; Endocrinology; (2006) 147: 2879-85.
Chang J C, Wu M C, Cheng J T.; Increase of insulin sensitivity by stevioside in fructose-rich chow-fed rats; Horm. Metab Res; (2005) 37: 610-16.
Edlund H. Transcribing pancreas; Diabetes; (1998) Dec; 47(12):1817-23.
Habener J F, Kemp D M, Thomas MK. Mini review: transscriptional regulation in pancreatic development; Endocrinology; (2005) 146: 1025-34.
Hansen, L., Urioste, S., Petersen, H. V., Jensen, J. N., Eiberg, H., Barbetti, F., Serup, P., Hansen, T., and Pedersen, O. Missense Mutations in the Human Insulin Promoter Factor-1 Gene and Their Relation to Maturity-Onset Diabetes of the Young and Late-Onset Type 2 Diabetes Mellitus in Caucasians; J. Clin. Endocrinol. Metab.; (2000) 85, 1323-1326.
Heding L G.; Determination of total serum insulin (IRI) in insulin-treated diabetic patients; Diabetologia; (1972) 8: 260-66.
Henseleit K D, Nelson S B, Kuhlbrodt K, Hennings J C, Ericson J, Sander M.; NKX6 transcription factor activity is required for alpha- and beta-cell development in the pancreas; Development; (2005) 132: 3139-49.
Hermansen, K.; Sondergaard, M.; Hoie, L.; Carstensen, M.; Brock, B.; Beneficial effects of a soy-based dietary supplement on lipid levels and cardiovascular risk markers in type 2 diabetic subjects. Diabetes Care; (2001) 24(2); 228-33.
Jeppesen P B, Gregersen S, Poulsen C R, Hermansen K.; Stevioside acts directly on pancreatic beta cells to secrete insulin: actions independent of cyclic adenosine monophosphate and adenosine triphosphate-sensitive K+-channel activity; Metabolism; (2000) 49: 208-14.
Mach F.; Inflammation is a crucial feature of atherosclerosis and a potential target to reduce cardiovascular events. Handb Exp Pharmacol. (2005);(170):697-722. Review
Malecki M T, Jhala U S, Antonelis A, et al.; Mutations in NEURODI1 are associated with the development of type 2 diabetes mellitus; Nat. Genet; (1999) 23: 223-28.
McGarry J.D.; Dysregulation of Fatty Acid Metabolism in the Etiology of Type 2 Diabetes. Diabetes; (2002) 51 (1): 7-18. PMID 11756317
Kruhoffer M, Jensen J L, Laiho P et al.; Gene expression signatures for colorectal cancer microsatetellite status and HNPCC; Br J. cancer (2005) 92: 2240-48.
Reusch J.E.B., and Klemm D.J.; Editorial: Nutrition and Fat Cell Differentiation. Endocrinology; (1999) Vol. 140, No. 7
Saltiel, A.R.; New Perspectives into the Molecular Pathogenesis and Treatment of Type 2 Diabetes. Cell; (2001), 104, 517-529
Storlien LH, Jenkins AB, Chisholm DJ, Pascoe WS, Khouri S, Kraegen EW; Influence of dietary fat composition on development of insulin resistance in rats. Relationship to muscle triglyceride and ω-3 fatty acids in muscle phospholipids. Diabetes (1991); 40:280-289.
Tolis G, Angelopoulos N G, Katounda E, et al.; Medical treatment of acromegaly: comorbidities and their reversiblility by smatostatin analogs; Neuroendocrinology; (2006) 83: 249-57.
Usala AL, Madigan T, Burguera B, Sinha MK, Caro JF, Cunningham P, et al.; Brief report: Treatment of insulin-resistant ketoacidosis with insulin-like growth factor I in an adolescent with insulin diabetes. N Engl J Med. (1992); 327:853-7.

### Further embodiments

Further embodiments of the invention include:
1. Use of a substance selected from the group consisting of isosteviol and steviol, or pharmaceutically acceptable salts, solvates or prodrugs thereof for the manufacture of a composition for the treatment of insulin resistance.
2. Use according to embodiment 1, wherein the substance is isosteviol, or pharmaceutically acceptable salts, solvates or prodrugs thereof.
3. Use according to any of embodiments 1 or 2, wherein the insulin resistance is in a mammal, such as e.g., a human subject.
4. Use according to any of embodiments 1-3, wherein the substance, or pharmaceutically acceptable salts, solvates or prodrugs thereof, is given to a human subject in a daily dosage in a range of from about 5 mg to about 1500 mg.
5. Use according to any of embodiments 1-4, wherein the substance, or pharmaceutically acceptable salts, solvates or prodrugs thereof, is given to a human subject in a daily dosage in a range of from about 5 mg to about 500 mg, such as e.g., from about 10 mg to about 500 mg, about 20 mg to about 500 mg, about 30 mg to about 500 mg, about 40 mg to about 500 mg, about 50 mg to about 500 mg, about 100 mg to about 500 mg, about 5 mg to about 400 mg, about 5 mg to about 300 mg, about 5 mg to about 200 mg, about 5 mg to about 100 mg, about 100 mg to about 500 mg, about 100 mg to about 400 mg, about 100 mg to about 300 mg, about 100 mg to about 200 mg, about 200 mg to about 500 mg, about 200 mg to about 400 mg, or about 200 mg to about 300 mg, or in a range of from about 500 mg to about 1000 mg, such as e.g., from about 500 mg to about 900 mg, about 500 mg to about 800 mg, about 500 mg to about 700 mg, about 500 mg to about 600 mg, about 600 mg to about 1000 mg, about 700 mg to about 1000 mg, about 800 mg to about 1000 mg, about 900 mg to about 1000 mg, or about 600 mg to about 900 mg, or in a range of from about 1000 mg to about 1500 mg, such as e.g., from about 1000 mg to about 1400 mg, about 1000 mg to about 1300 mg, about 1000 mg to about 1200 mg, about 1000 mg to about 1100 mg, about 1100 mg to about 1500 mg, about 1200 mg to about 1500 mg, about 1300 mg to about 1500 mg, about 1400 mg to about 1500 mg, or about 1100 mg to about 1400 mg.
6. Use according to any of claims 1-5, wherein the medicament further comprises one or more additional active substance.
7. Use according to any of claims 1-6, wherein the medicament is for oral, peroral, sublingual, parenteral, intramuscular, topical, buccal, nasal, or inhalation administration; preferably for oral administration.

## Claims

1. Use of a substance selected from the group consisting of isosteviol and steviol, or pharmaceutically acceptable salts, solvates or prodrugs thereof, for the manufacture of a composition for the treatment of insulin resistance or diseases associated with insulin resistance in a human subject, wherein the substance is given in a daily dosage in a range of from about 5 mg to about 1500 mg.

2. Use according to claim 1, wherein the composition is for the treatment of insulin resistance.

3. Use according to any of the preceding claims, wherein the substance is isosteviol, or pharmaceutically acceptable salts, solvates or prodrugs thereof.

4. Use according to any of claims 1-3, wherein the disease associated with insulin resistance is selected from the group consisting of Type 2 diabetes mellitus, insulin resistance syndrome, impaired glucose tolerance, the metabolic syndrome, hyperglycemia, hyperinsulinemia, arteriosclerosis, hypercholesterolemia, hypertriglyceridemia, hyperlipidemia, dyslipidemia, obesity, central obesity, polycystic ovarian syndrome, hypercoagulability, hypertension, microalbuminuria, and any combinations thereof.

5. Use according to claim 4, wherein the disease is selected from the group consisting of hypercholesterolemia, hypertriglyceridemia, hyperlipidemia, dyslipidemia, hypertension, microalbuminuria, hypercoagulability, polycystic ovarian syndrome, obesity, central obesity and combinations thereof.

6. Use according to any of the preceding claims, wherein the substance is given in a daily dosage in a range of from about 5 mg to about 500 mg, such as e.g., from about 10 mg to about 500 mg, about 20 mg to about 500 mg, about 30 mg to about 500 mg, about 40 mg to about 500 mg, about 50 mg to about 500 mg, about 100 mg to about 500 mg, about 5 mg to about 400 mg, about 5 mg to about 300 mg, about 5 mg to about 200 mg, about 5 mg to about 100 mg, about 100 mg to about 500 mg, about 100 mg to about 400 mg, about 100 mg to about 300 mg, about 100 mg to about 200 mg, about 200 mg to about 500 mg, about 200 mg to about 400 mg, or about 200 mg to about 300 mg.

7. Use according to any of claims 1-5, wherein the substance is given in a daily dosage in a range of from about 500 mg to about 1000 mg, such as e.g., from about 500 mg to about 900 mg, about 500 mg to about 800 mg, about 500 mg to about 700 mg, about 500 mg to about 600 mg, about 600 mg to about 1000 mg, about 700 mg to about 1000 mg, about 800 mg to about 1000 mg, about 900 mg to about 1000 mg, or about 600 mg to about 900 mg.

8. Use according to any of claims 1-5, wherein the substance is given in a daily dosage in a range of from about 1000 mg to about 1500 mg, such as e.g., from about 1000 mg to about 1400 mg, about 1000 mg to about 1300 mg, about 1000 mg to about 1200 mg, about 1000 mg to about 1100 mg, about 1100 mg to about 1500 mg, about 1200 mg to about 1500 mg, about 1300 mg to about 1500 mg, about 1400 mg to about 1500 mg, or about 1100 mg to about 1400 mg.

9. Use according to any of the preceding claims, wherein the medicament further comprises one or more additional active substance.

10. Use according to any of the preceding claims, wherein the medicament is for oral, peroral, sublingual, parenteral, intramuscular, topical, buccal, nasal, or inhalationadministration; preferably oral administration.

11. Use according to any of the preceding claims, wherein the steviol or isosteviol is isolated from a plant source.
